(19) Europäisches Patentamt
European Patent Office
Office européen des brevets

(11) **EP 4 685 813 A2**

(12) **EUROPEAN PATENT APPLICATION**

(43) Date of publication:
**28.01.2026 Bulletin 2026/05**

(51) International Patent Classification (IPC):
***G16H 50/50*** (2018.01)

(21) Application number: **25222412.6**

(22) Date of filing: **01.11.2022**

(52) Cooperative Patent Classification (CPC):
**G16H 20/17; G16H 50/50**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.11.2021 US 202163263433 P**

(62) Document number(s) of the earlier application(s) in accordance with Art. 76 EPC:
**22818576.5 / 4 427 234**

(71) Applicants:
• **DexCom, Inc.**
**San Diego, CA 92121 (US)**
• **University of Padova**
**35122 Padua (IT)**

(72) Inventors:
• **FACCIOLI, Simone**
**San Diego, California, 92121 (US)**

• **FACCHINETTI, Andrea**
**San Diego, California, 92121 (US)**
• **DEL FAVERO, Simone**
**San Diego, California, 92121 (US)**
• **PRENDIN, Francisco**
**San Diego, California, 92121 (US)**
• **SPARACINO, Giovanni**
**San Diego, California, 92121 (US)**

(74) Representative: **Crowell & Moring U.K. LLP**
**199 Bishopsgate**
**London EC2M 3TY (GB)**

Remarks:
This application was filed on 10.12.2025 as a divisional application to the application mentioned under INID code 62.

(54) **PREDICTION FUNNEL FOR GENERATION OF HYPO- AND HYPER GLYCEMIC ALERTS BASED ON CONTINUOUS GLUCOSE MONITORING DATA**

(57) Certain aspects of the present disclosure relate to methods and systems for providing decision support around glucose management for patients with diabetes. Time-varying inputs including blood glucose, meal intake information, and amount of infused insulin are processed using a machine learning model to obtain predicted glucose levels for a plurality of prediction horizons and uncertainties for the predictions. A confidence interval is generated for each prediction and the confidence intervals are compared to hypo- and hyperglycemic thresholds. If a confidence interval is entirely below or entirely above the hypo- and hyperglycemic thresholds, respectively, then a decision support output is provided.

FIG. 7

**EP 4 685 813 A2**

**Description**

**CROSS-REFERENCE TO RELATED APPLICATIONS**

This application claims priority to and benefit of U.S. Provisional Patent Application No. 63/263,433, filed November 2, 2021, which is hereby assigned to the assignee hereof and hereby expressly incorporated by reference in its entirety as if fully set forth below and for all applicable purposes.

**BACKGROUND**

The technology advancements provided by continuous glucose monitoring (CGM) devices [1] and portable pumps for continuous subcutaneous insulin infusion (CSII) [2] have considerably improved the quality of life for subjects with type 1 diabetes (T1D). As pointed out in a recent report on artificial intelligence (AI) applications for diabetes management [3], the combined use of CGM devices, insulin pumps and dedicated mobile applications [4] brought the possibility of recording different types of information, for instance: CGM data, insulin, meal, physical activity, and self-reported life events. This information enables the development of advanced AI-enabled decision support systems (DSSs), which are composite tools that implement multiple software modules to support the patient in the decision-making process.
One of the key elements that can be embedded in an advanced DSS is the prediction module. In fact, knowing ahead of time if blood glucose (BG) is getting close to possibly harmful values allows patients to take proactive actions to mitigate or avoid critical episodes like hypoglycemia (i.e., BG below 70 mg/dL), considerably improving T1D management [5]-[10]. Several research efforts have investigated BG prediction [11], and a number of literature studies have focused on the challenge of forecasting hypoglycemic episodes [12]. In these efforts and studies, hypoglycemia prediction was addressed either by classification-based or regression-based approaches. Classification-based approaches consist of developing a binary classifier [13], i.e., an algorithm producing only two types of possible output, "impending hypoglycemia" or "no hypoglycemia predicted." Regression-based approaches, instead, are two-step procedures that as a first step predict the future glucose concentration via regression, and then raise an alarm if the predicted value falls below a suitable threshold (usually, but not necessarily, 70 mg/dL). In these efforts and studies, predicted glucose concentrations used in the first step of the regression-based approach were obtained by using either linear predictors [14]-[16] or non-linear approaches [17]-[23]. Interestingly, the superiority of one approach over the others has not yet been demonstrated.

**BRIEF DESCRIPTION OF THE DRAWINGS**

So that the manner in which the above-recited features of the present disclosure can be understood in detail, a more particular description, briefly summarized above, may be had by reference to aspects, some of which are illustrated in the drawings. It is to be noted, however, that the appended drawings illustrate only certain typical aspects of this disclosure and are therefore not to be considered limiting of its scope, for the description may admit to other equally effective aspects.

FIG. 1 illustrates aspects of an example decision support system that may be used in connection with implementing embodiments of the present disclosure.

FIG. 2 is a diagram conceptually illustrating an example continuous analyte monitoring system including example continuous analyte sensor(s) with sensor electronics, in accordance with certain aspects of the present disclosure.

FIG. 3 illustrates example inputs used by a prediction module of the decision support system of FIG. 1, according to some embodiments disclosed herein.

FIG. 4 is an example workflow for training a machine learning model to predict future glucose values, according to some embodiments disclosed herein.

FIG. 5A illustrates a Clarke error grid, according to some embodiments disclosed herein.

FIG. 5B illustrates an example penalty function based on the Clarke error grid, according to some embodiments disclosed herein.

Fig. 5C illustrates example glucose predictions for a machine learning model trained using glucose specific mean squared error, according to some embodiments disclosed herein.

FIG. 6 is an example workflow illustrating the use of a prediction funnel, according to certain embodiments of the present disclosure.

Fig. 7 is an example flow diagram depicting a method for generating alerts using the prediction funnel of FIG. 6, according to certain embodiments of the present disclosure.

FIG. 8 is an example plot showing the operation of the prediction funnel, according to certain embodiments of the present disclosure.

FIG. 9 is an example workflow illustrating use of the prediction funnel to control an automatic insulin delivery (AID) system, according to some embodiments disclosed herein.

FIG. 10 is a block diagram depicting a computing device configured to perform the operations of FIGs. 6, 7, and/or 8, according to certain embodiments disclosed herein.

To facilitate understanding, identical reference numerals have been used, where possible, to designate identical elements that are common to the figures. It is contemplated that elements disclosed in one aspect may be beneficially utilized on other aspects without specific recitation.

## DETAILED DESCRIPTION

The following description details a machine-learning-based approach to predicting hypoglycemic and/or hyperglycemic events based on glucose predictions generated using a glucose prediction model, such as an individualized glucose prediction model. Using an individualized prediction model enables the handling of large inter- and intra-subject variability, which is one of the major challenges in glucose prediction. The glucose prediction model may be a linear prediction model, which enables a high degree of individualization. A linear prediction model provides powerful convergence results and enables statistical properties analysis [24]. Moreover, a linear prediction model may be used with computationally convenient algorithms, such the Kalman predictor [25]. Finally, although the metabolic physiology is non-linear [26], linear strategies have proved to be able to capture the essential dynamics [14]-[16], [27], [28], and remain challenging competitors to non-linear approaches [29], [30].

The following detailed description provides an improved approach for both (a) training a prediction model to predict hypo- and hyperglycemia and (b) an alarm-raising strategy (e.g., a rule-based model) that takes as an input the output of the prediction model. The prediction model may be trained using a cost function, previously introduced in [31], which is able to take into account the clinical impact of the prediction error, thus enabling the identification of more effective models for predicting future hypoglycemic episodes. The alarm raising strategy does not focus on a single prediction horizon, but rather simultaneously considers multiple prediction horizons, thereby accounting for the expected decrease of accuracy in predictions from the prediction model as the prediction horizon increases.

*Example Decision Support System Including a Prediction Module*

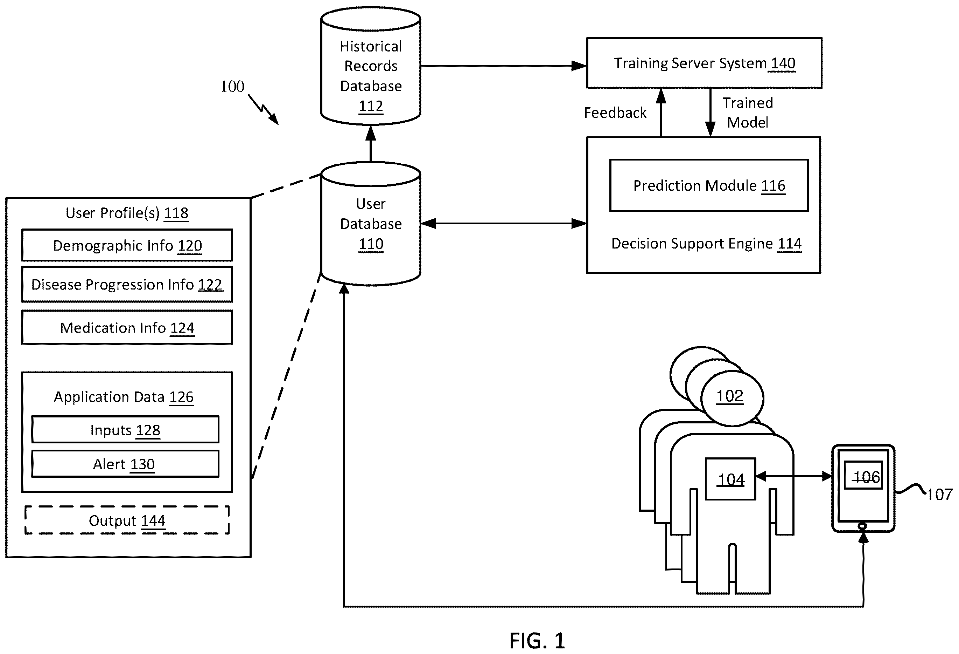

FIG. 1 illustrates an example decision support system 100 for implementing the improved prediction model training approach and the improved alarm-raising strategy, in accordance with certain embodiments. The decision support system 100 is configured to provide decision support to users 102 (individually referred to herein as a user and collectively referred to herein as users), using sensor data provided by a continuous analyte monitoring system 104, including, at least, a continuous glucose sensor. A user, in certain embodiments, may be the patient or, in some cases, the patient's caregiver. In certain embodiments, decision support system 100 includes continuous analyte monitoring system 104, a display device 107 that executes application 106, a decision support engine 114, a user database 110, a historical records database 112, a training server system 140, and a decision support engine 114, each of which is described in more detail below. The training server system 140 may implement the improved approach for training a prediction model, whereas a prediction module 116 within the decision support engine 114 implements the improved alarm-raising strategy using the prediction model.

The term "analyte" as used herein is a broad term used in its ordinary sense, including, without limitation, to refer to a substance or chemical constituent in a biological fluid (for example, blood, interstitial fluid, cerebral spinal fluid, lymph fluid or urine) that can be analyzed. In the examples described below, the analyte is glucose in the blood stream of the user. However, the concentration of any analyte or any time-varying value that can be measured may be predicted using the approach described herein. For example, analytes can include naturally occurring substances, artificial substances, metabolites, and/or reaction products. Analytes for measurement by the devices and methods may include, but may not be limited to, potassium, glucose, acarboxyprothrombin; acylcarnitine; adenine phosphoribosyl transferase; adenosine deaminase; albumin; alpha-fetoprotein; amino acid profiles (arginine (Krebs cycle), histidine/urocanic acid, homocysteine, phenylalanine/tyrosine, tryptophan); androstenedione; antipyrine; arabinitol enantiomers; arginase; benzoylecgonine (cocaine); biotinidase; biopterin; c-reactive protein; carnitine; carnosinase; CD4; ceruloplasmin; chenodeoxycholic acid; chloroquine; cholesterol; cholinesterase; conjugated 1-β hydroxy-cholic acid; cortisol; creatine kinase; creatine kinase MM isoenzyme; cyclosporin A; d-penicillamine; de-ethylchloroquine; dehydroepiandrosterone sulfate; DNA (acetylator polymorphism, alcohol dehydrogenase, alpha 1-antitrypsin, glucose-6-phosphate dehydrogenase, hemoglobin A, hemoglobin S, hemoglobin C, hemoglobin D, hemoglobin E, hemoglobin F, D-Punjab, hepatitis B virus, HCMV, HIV-1, HTLV-1, MCAD, RNA, PKU, Plasmodium vivax, 21-deoxycortisol); desbutylhalofantrine; dihydropteridine reductase; diptheria/tetanus antitoxin; erythrocyte arginase; erythrocyte protoporphyrin; esterase D; fatty acids/acylglycines; free β-human chorionic gonadotropin; free erythrocyte porphyrin; free thyroxine (FT4); free tri-iodothyronine (FT3); fumarylacetoacetase; galactose/gal-1-phosphate; galactose-1-phosphate uridyltransferase; gentamicin; glucose-6-phosphate dehydrogenase; glutathione; glutathione perioxidase; glycocholic acid; glycosylated hemoglobin; halofantrine; hemoglobin variants; hexosaminidase A; human erythrocyte carbonic anhydrase I; 17-alpha-hydroxyprogesterone; hypoxanthine phosphoribosyl transferase; immunoreactive trypsin; lactate; lead; lipoproteins ((a), B/A-1, β); lysozyme; mefloquine; netilmicin; phenobarbitone; phenytoin; phytanic/pristanic acid; progesterone; prolactin; prolidase; purine nucleoside phosphorylase; quinine; reverse tri-iodothyronine (rT3); selenium; serum pancreatic lipase; sisomicin; somatomedin C; specific antibodies recognizing any one or more of the following that may include (adenovirus, anti-nuclear antibody, anti-zeta antibody, arbovirus, Aujeszky's disease virus, dengue virus, Dracunculus medinensis, Echinococcus granulosus, Entamoeba histolytica, enterovirus, Giardia duodenalisa, Helicobacter pylori, hepatitis B virus, herpes virus, HIV-1, IgE (atopic disease), influenza virus, Leishmania donovani, leptospira, measles/mumps/rubella, Mycobacterium leprae, Mycoplasma pneumoniae, Myoglobin, Onchocerca volvulus, parainfluenza virus, Plasmodium falciparum, poliovirus, Pseudomonas aeruginosa, respiratory syncytial virus, rickettsia (scrub typhus), Schistosoma mansoni, Toxoplasma gondii, Trepenoma pallidium, Trypanosoma cruzi/rangeli, vesicular stomatis virus, Wuchereria bancrofti, yellow fever virus); specific antigens (hepatitis B virus, HIV-1); succinylacetone; sulfadoxine; theophylline; thyrotropin (TSH); thyroxine (T4); thyroxine-binding globulin; trace elements; transferrin; UDP-galactose-4-epimerase; urea; uroporphyrinogen I synthase; vitamin A; white blood cells; and zinc protoporphyrin. Salts, sugar, protein, fat, vitamins, and hormones naturally occurring in blood or interstitial fluids can also constitute analytes in certain implementations. Ions are a charged atom or compounds that may include the following (sodium, potassium, calcium, chloride, nitrogen, or bicarbonate, for example). The analyte can be naturally present in the biological fluid, for example, a metabolic product, a hormone, an antigen, an antibody, an ion and the like. Alternatively, the analyte can be introduced into the body or exogenous, for example, a contrast agent for imaging, a radioisotope, a chemical agent, a fluorocarbon-based synthetic blood, a challenge agent analyte (e.g., introduced for the purpose of measuring the increase and or decrease in rate of change in concentration of the challenge agent analyte or other analytes in response to the introduced challenge agent analyte), or a drug or pharmaceutical composition, including but not limited to exogenous insulin; glucagon, ethanol; cannabis (marijuana, tetrahydrocannabinol, hashish); inhalants (nitrous oxide, amyl nitrite, butyl nitrite, chlorohydrocarbons, hydrocarbons); cocaine (crack cocaine); stimulants (amphetamines, methamphetamines, Ritalin, Cylert, Preludin, Didrex, PreState, Voranil, Sandrex, Plegine); depressants (barbiturates, methaqualone, tranquilizers such as Valium, Librium, Miltown, Serax, Equanil, Tranxene); hallucinogens (phencyclidine, lysergic acid, mescaline, peyote, psilocybin); narcotics (heroin, codeine, morphine, opium, meperidine, Percocet, Percodan, Tussionex, Fentanyl, Darvon, Talwin, Lomotil); designer drugs (analogs of fentanyl, meperidine, amphetamines, methamphetamines, and phencyclidine, for example, Ecstasy); anabolic steroids; and nicotine The metabolic products of drugs and pharmaceutical compositions are also contemplated analytes. Analytes such as neurochemicals and other chemicals generated within the body can also be analyzed, such as, for example, ascorbic acid, uric acid, dopamine, noradrenaline, 3-methoxytyramine (3MT), 3,4-Dihydroxyphenylacetic acid (DOPAC), Homovanillic acid (HVA), 5-Hydroxytryptamine (5HT), and 5-Hydroxyindoleacetic acid (FHIAA), and intermediaries in the Citric Acid Cycle.

In certain embodiments, continuous analyte monitoring system 104 is configured to continuously measure one or more analytes and transmit the analyte measurements to an electric medical records (EMR) system (not shown in FIG. 1). An EMR system is a software platform which allows for the electronic entry, storage, and maintenance of digital medical data. An EMR system is generally used throughout hospitals and/or other caregiver facilities to document clinical information on patients over long periods. EMR systems organize and present data in ways that assist clinicians with, for example, interpreting health conditions and providing ongoing care, scheduling, billing, and follow up. Data contained in an EMR system may also be used to create reports for clinical care and/or disease management for a patient. In certain embodiments, the EMR may be in communication with decision support engine 114 (e.g., via a network) for performing the techniques described herein. The communication could come through a variety of network connection data configurations including but not limited to web API protocols, HL7, FHIR, EDI, XML, CDA, and others.

These data communication configurations could be sent directly to the EMR, or through one or more intermediary systems including but not limited to an interface engine before entering the EMR system to then be displayed. Patient data communicated into the EMR via any of these other means could be matched to a patient record through probabilistic matching, manual human matching, or an EMPI or MPI (master patient index, electronic master patient index) to ensure that the data input to one system matches the patient information in another system. Data from an analyte device could also be matched with data from alternative devices or systems prior to being inputted into the EMR or data could be sent in the reverse direction into our historical records database 112, user database 110, and or decision support engine.

These data transfers allow the system to perform optimized decision support through the means described herein. In particular, as described herein, decision support engine 114 may obtain data associated with a user, use the obtained data as input into one or more trained model(s), and output a prediction. In some cases, the EMR may provide the data to decision support engine 114 to be used as input into the one or more models. Further, in some cases, decision support engine 114, after making a prediction, may provide the prediction to the EMR.

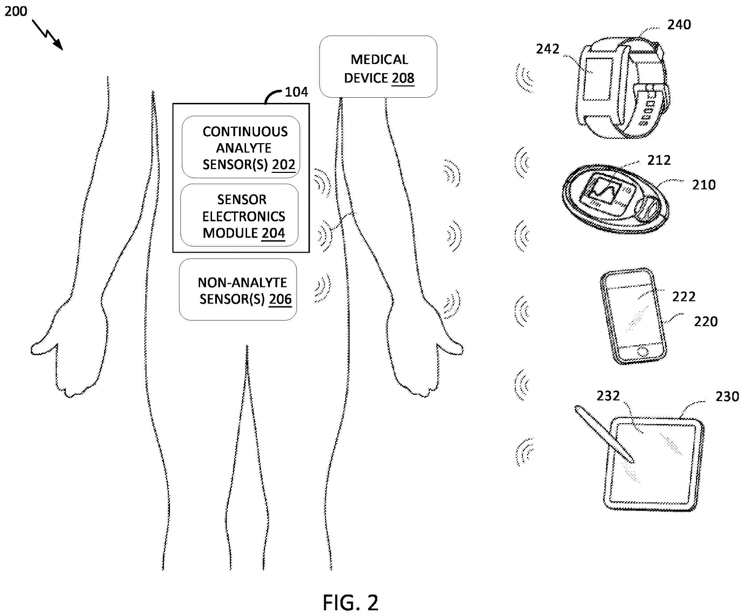

In certain embodiments, continuous analyte monitoring system 104 is configured to continuously measure one or more analytes and transmit the analyte measurements to display device 107 for use by application 106. In some embodiments, continuous analyte monitoring system 104 transmits the analyte measurements to display device 107 through a wireless connection (e.g., Bluetooth connection). In certain embodiments, display device 107 is a smart phone. However, in certain other embodiments, display device 107 may instead be any other type of computing device such as a laptop computer, a smart watch, a tablet, or any other computing device capable of executing application 106. In some embodiments, continuous analyte monitoring system 104 and/or analyte sensor application 106 transmit the analyte measurements to one or more other individuals having an interest in the health of the patient (e.g., a family member or physician for real-time treatment and care of the patient). Continuous analyte monitoring system 104 may be described in more detail with respect to FIG. 2.

Application 106 is a mobile health application that is configured to receive and analyze analyte measurements from analyte monitoring system 104. In particular, application 106 stores information about a user, including the user's analyte measurements, in a user profile 118 associated with the user for processing and analysis, as well as for use by decision support engine 114 to provide decision support outputs (e.g., alerts, recommendations, guidance, signals to control insulin pumps/pens, etc.) to the user.

Decision support engine 114 refers to a set of software instructions with one or more software modules, including the prediction module 116. In certain embodiments, decision support engine 114 executes entirely on one or more computing devices in a private or a public cloud. In such embodiments, application 106 communicates with decision support engine 114 over a network (e.g., Internet). In some other embodiments, decision support engine 114 executes partially on one or more local devices, such as display device 107, and partially on one or more computing devices in a private or a public cloud. In some other embodiments, decision support engine 114 executes entirely on one or more local devices, such as display device 107. As discussed in more detail herein, decision support engine 114 may provide decision support outputs to the user via application 106. Decision support engine 114 provides decision support outputs based on information included in user profile 118.

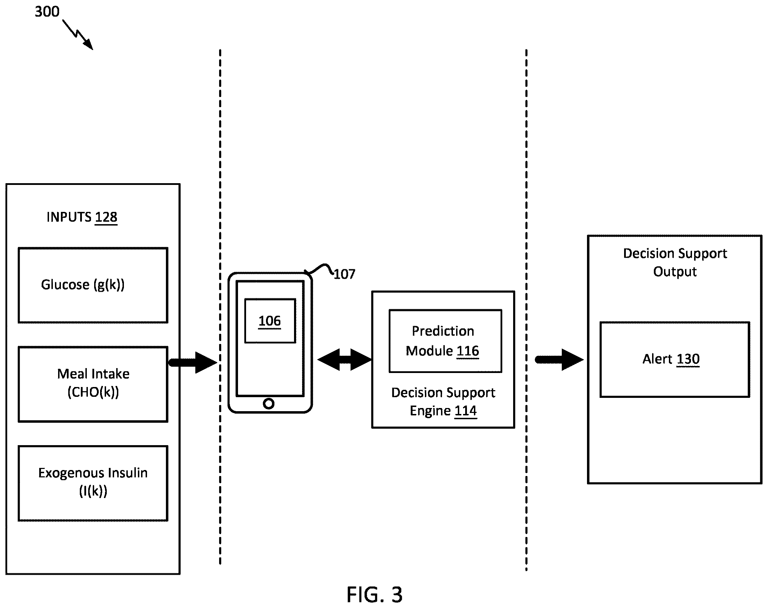

User profile 118 may include information collected about the user from application 106. For example, application 106 provides a set of inputs 128, including the analyte measurements received from continuous analyte monitoring system 104, that are stored in user profile 118. In certain embodiments, inputs 128 provided by application 106 include other data in addition to analyte measurements received from continuous analyte monitoring system 104. For example, application 106 may obtain additional inputs 128 through manual user input, one or more other non-analyte sensors or devices, other applications executing on display device 107, etc. Non-analyte sensors and devices include one or more of, but are not limited to, an insulin pump, an electrocardiogram (ECG) sensor or heart rate monitor, a blood pressure sensor, a sweat sensor, a respiratory sensor, a thermometer, sensors or devices provided by display device 107 (e.g., accelerometer, camera, global positioning system (GPS), heart rate monitor, etc.) or other user accessories (e.g., a smart watch), or any other sensors or devices that provide relevant information about the user. Inputs 128 of user profile 118 provided by application 106 are described in further detail below with respect to FIG. 3.

The prediction module 116 of decision support engine 114 is configured to process the set of inputs 128 to predict future analyte levels, risk of adverse events (e.g., hypo- and hyperglycemia), and/or determine whether or not to provide a decision support output and the content of such output, based on the inputs 128.. As described below, various types of decision support outputs may be provided, such as alerts, decision support recommendations, control signals for controlling the operations of a medicament delivery device (e.g., insulin pump or pen), etc.

User profile 118 may also include demographic info 120, disease progression info 122, and/or medication info 124. In certain embodiments, such information may be provided through user input or obtained from certain data stores (e.g., electronic medical records (EMRs), etc.). In certain embodiments, demographic info 120 may include one or more of the user's age, body mass index (BMI), ethnicity, gender, etc. In certain embodiments, disease progression info 122 may include information about a disease of a user, such as diabetes, or whether the user has a history of hyperkalemia, hypokalemia, hyperglycemia, hypoglycemia, etc. In certain embodiments, information about a user's disease may also include the length of time since diagnosis, the stage of disease, the level of disease control, level of compliance with disease management therapy, other types of diagnosis (e.g., heart disease, obesity) or measures of health (e.g., heart rate, exercise, stress, sleep, etc.), and/or the like. In certain embodiments, disease progression info 122 may be provided as an output of one or more predictive algorithms and/or trained models based on analyte sensor data generated, for example, through continuous analyte monitoring system 104.

In certain embodiments, medication info 124 may include information about the amount, frequency, and type of a medication taken by a user. In certain embodiments, the amount, frequency, and type of a medication taken by a user is time-stamped and correlated with the user's analyte levels, thereby, indicating the impact the amount, frequency, and type of the medication had on the user's analyte levels. In certain embodiments, medication information 124 may include information about consumption of one or more drugs known to control and/or improve glucose homeostasis. One or more drugs known to control and/or improve glucose homeostasis may include medications to lower blood glucose levels such as insulin, including rapid acting, and long-acting insulin, other glucose lowering medications, such as metformin, and the like. As described in more detail below, decision support system 100 may be configured to use medication information 124 to determine optimal insulin administration to be prescribed to different users. In particular, decision support system 100 may be configured to identify one or more optimal insulin administration based on the health of the patient, the patient's current condition, and/or effectiveness of insulin administration.

In certain embodiments, user profile 118 is dynamic because at least part of the information that is stored in user profile 118 may be revised over time and/or new information may be added to user profile 118 by decision support engine 114 and/or application 106. Accordingly, information in user profile 118 stored in user database 110 provides an up-to-date repository of information related to a user.

User database 110, in some embodiments, refers to a storage server that operates in a public or private cloud. User database 110 may be implemented as any type of datastore, such as relational databases, non-relational databases, key-value datastores, file systems including hierarchical file systems, and the like. In some exemplary implementations, user database 110 is distributed. For example, user database 110 may comprise a plurality of persistent storage devices, which are distributed. Furthermore, user database 110 may be replicated so that the storage devices are geographically dispersed.

User database 110 includes user profiles 118 associated with a plurality of users who similarly interact with application 106 executing on the display devices 107 of the other users. User profiles stored in user database 110 are accessible to not only application 106, but decision support engine 114, as well. User profiles in user database 110 may be accessible to application 106 and decision support engine 114 over one or more networks (not shown). As described above, decision support engine 114, and more specifically prediction module 116 of decision support engine 114, can fetch inputs 128 from user database 110 and generate decision support outputs, which can then be stored as application data 126 in user profile 118.

In certain embodiments, user profiles 118 stored in user database 110 may also be stored in historical records database 112. User profiles 118 stored in historical records database 112 may provide a repository of up-to-date information and historical information for each user of application 106. Thus, historical records database 112 essentially provides all data related to each user of application 106, where data is stored according to an associated timestamp. The timestamp associated with information stored in historical records database 112 may identify, for example, when information related to a user has been obtained and/or updated.

Further, historical records database 112 may maintain time series data collected for users over a period of time, including for users who use continuous analyte monitoring system 104 and application 106. For example, analyte data for a user who has used continuous analyte monitoring system 104 and application 106 for a period of five years to manage the user's health may have time series analyte data associated with the user maintained over the five-year period.

Further, in certain embodiments, historical records database 112 may include data for one or more patients who are not users of continuous analyte monitoring system 104 and/or application 106. For example, historical records database 112 may include information (e.g., user profile(s)) related to one or more patients analyzed by, for example, a healthcare physician (or other known method), and not previously diagnosed with diabetes, as well as information (e.g., user profile(s)) related to one or more patients who were analyzed by, for example, a healthcare physician (or other known method) and were previously diagnosed with (varying types and stages of) diabetes. Data stored in historical records database 112 may be referred to herein as population data.

Data related to each patient stored in historical records database 112 may provide time series data collected over the disease lifetime of the patient, wherein the disease may be diabetes. For example, the data may include information about the patient prior to being diagnosed with diabetes and information associated with the patient during the lifetime of the disease, including information related to diseases that are co-morbid in relation to diabetes. Such information may indicate symptoms of the patient, physiological states of the patient, glucose levels of the patient, insulin levels of the patient, states/conditions of one or more organs of the patient, habits of the patient (e.g., activity levels, food consumption, etc.), medication prescribed, etc.

In another example, the data may include information about the patient prior to being diagnosed with diabetes, hyperglycemia, or hypoglycemia and information associated with the patient during the lifetime of the disease, including information related to diabetes as it progressed and/or regressed in the patient, as well as information related to other diseases, such as hyperglycemia, hypoglycemia, kidney disease, hypertension, heart conditions and diseases, or similar diseases that are co-morbid in relation to diabetes. Such information may indicate symptoms of the patient, physiological states of the patient, glucose levels of the patient, potassium levels of the patient, lactate levels of patient, insulin levels of the patients, states/conditions of one or more organs of the patient, habits of the patient (e.g., activity levels, food consumption, etc.), medication prescribed, medication adherence, etc., throughout the lifetime of the disease.

Although depicted as separate databases for conceptual clarity, in some embodiments, user database 110 and historical records database 112 may operate as a single database. That is, historical and current data related to users of continuous analyte monitoring system 104 and application 106, as well as historical data related to patients that were not previously users of continuous analyte monitoring system 104 and application 106, may be stored in a single database. The single database may be a storage server that operates in a public or private cloud.

As mentioned previously, decision support system 100 is configured to diagnose, stage, treat, and assess risks of diabetes for a user using continuous analyte monitoring system 104, including, at least, a continuous glucose sensor. For example, decision support engine 114 may be configured to collect information associated with a user in user profile 118 stored in user database 110, to perform analytics thereon to (1) predict future analyte levels (e.g., glucose levels), (2) predict risk of adverse events, such as hypoglycemic and hyperglycemia, (3) generate alarms based on the predicted risk of the adverse events, and/or (4) provide treatment recommendations. In certain embodiments, a user's glucose metrics may include glucose levels, glucose level rate(s) of change, glucose trend(s), mean glucose, glucose management indicator (GMI), glycemic variability, time in range (TIR), glucose clearance rate, etc.

User profile 118 may be accessible to decision support engine 114 over one or more networks (not shown) for performing such analytics. In certain embodiments, decision support engine 114 is configured to provide real-time and/or non-real-time decision support around diabetes to the user and/or others, including but not limited, to healthcare providers (HCP), family members of the user, caregivers of the user, researchers, and/or other individuals, systems, and/or groups supporting care or learning from the data.

In certain embodiments, decision support engine 114 may utilize a prediction module 116 including one or more trained machine learning models for (1) predicting future analyte levels (e.g., glucose levels), (2) predicting risk of adverse events, such as hypoglycemic and hyperglycemia, and/or (3) generating decision support outputs based on the predicted future analyte levels and/or the predicted risk of the adverse events. In the illustrated embodiment of FIG. 1, the prediction module 116 may utilize trained machine learning model(s) provided by a training server system 140. Although depicted as a separate server for conceptual clarity, in some embodiments, training server system 140 and decision support engine 114 may operate as a single server. That is, the model may be trained and used by a single server, or may be trained by one or more servers and deployed for use on one or more other servers. In certain embodiments, the model may be trained on one or many virtual machines (VMs) running, at least partially, on one or many physical servers in relational and/or non-relational database formats.

Training server system 140 is configured to train the machine learning model(s) using training data, which may include data (e.g., from user profiles) associated with one or more patients (e.g., users or non-users of continuous analyte monitoring system 104 and/or application 106) previously diagnosed with (1) being non diabetic or (2) varying stages of diabetes. The training data may be stored in historical records database 112 and may be accessible to training server system 140 over one or more networks (not shown) for training the machine learning model(s). The training data may also, in some cases, include user-specific data for a user over time.

The training data refers to a dataset that, for example, has been featurized and labeled. In certain embodiment, the dataset may be a population-based dataset, including a plurality of historical data records. Each historical data record in the dataset may include information corresponding to a different user profile stored in user database 110. Further, each historical data record may be featurized and labeled. In machine learning and pattern recognition, a feature is an individual measurable property or characteristic. Generally, the features that best characterize the patterns in the data are selected to create predictive machine learning models. Data labeling is the process of adding one or more meaningful and informative labels to provide context to the data for learning by the machine learning model.

As an illustrative example, each relevant characteristic of a user, which is reflected in a corresponding historical data record, may be a feature used in training the machine learning model. Such features may include features associated with the user's demographic information (e.g., age, gender, etc.), time-stamped analyte measurements (e.g., time-stamped glucose measurements), time-stamped meal intake information, time-stamped insulin administration information (e.g., insulin dosage administered). Each historical data record may also be labeled depending on what a corresponding model is being trained to predict.

The model(s) are then trained by training server system 140 using the featurized and labeled training data. In certain embodiments, the features of each historical data record may be used as input into the machine learning model(s), and the generated output may be compared to label(s) associated with the corresponding historical data record. The model(s) may compute a loss based on the difference between the generated output and the provided label(s). This loss is then used to modify the internal parameters or weights of the model. By iteratively processing each historical data record corresponding to each historical patient, in certain embodiments, the model(s) may be iteratively refined to generate accurate predictions.

As illustrated in FIG. 1, training server system 140 deploys these trained model(s) to decision support engine 114 for use during runtime. For example, decision support engine 114 may obtain user profile 118 associated with a user, use information in user profile 118 as input into the trained model(s), and output a prediction of future analyte levels (e.g., glucose levels) and/or risk of hypo- and/or hyperglycemia. The decision support engine 114 may then use the prediction to generate a decision support output. For example, in cases where the prediction is indicative of a hypo- or hyperglycemia event, the decision support output may include an alert (i.e., alarm) generated using an alert-raising model as described below. Generating an alert may include causing a display device (e.g., display device 107) to output a human-perceptible alert, such as a visible message, audible alert, or palpable alert (e.g., with a haptic device) that, e.g., may be indicative of the predicted event and/or the patient's glucose levels.

In certain embodiments, along with or instead of an alert, the decision support output may also include a decision support recommendation for the patient to engage in exercise, consume carbohydrates, administer insulin (e.g., alter the infusion rate), etc. In certain embodiments, along with or instead of an alert, the decision support output may further include a signal to an insulin pump to alter the amount of insulin that is being or to be administered to a user.

The decision support output may be provided to the user (e.g., through application 106), to a user's caretaker (e.g., a parent, a relative, a guardian, a teacher, a nurse, etc.), to a user's physician, or any other individual that has an interest in the wellbeing of the user for purposes of improving the user's health, such as, in some cases by effectuating the recommended treatment.

In certain embodiments, the user's own data is used to personalize the one or more models that may be initially trained based on population data. For example, a model (e.g., trained using population data) may be deployed for use by decision support engine 114 to predict future analyte levels for a user. After making a prediction using the model, decision support engine 114 may be configured to obtain the user's actual analyte values and compute a loss between the prediction and the actual glucose values, which can be used for retraining the model. Accordingly, the model may continue to be retrained and personalized using the computed loss between the prediction and the actual glucose values as input into the model to personalize the model for the user. Additional details regarding training the one or more models herein are described in further detail below.

FIG. 2 illustrates an analyte monitoring system 200 including an example continuous analyte sensor system 104, non-analyte sensor(s) 206, medical device 208, and a plurality of display devices 210, 220, 230, and 240, in accordance with certain aspects of the present disclosure. The components of the analyte monitoring system 200 is configured to operate continuously monitor one or more analytes of a user, in accordance with certain aspects of the present disclosure.

Continuous analyte monitoring system 104 in the illustrated embodiment includes sensor electronics module 204 and one or more continuous analyte sensor(s) 202 (individually referred to herein as continuous analyte sensor 202 and collectively referred to herein as continuous analyte sensors 202) associated with sensor electronics module 204. Sensor electronics module 204 may be in wireless communication (e.g., directly or indirectly) with one or more of display devices 210, 220, 230, and 240. In certain embodiments, sensor electronics module 204 may also be in wireless communication (e.g., directly or indirectly) with one or more medical devices, such as medical devices 208 (individually referred to herein as medical device 208 and collectively referred to herein as medical devices 208), and/or one or more other non-analyte sensors 206 (individually referred to herein as non-analyte sensor 206 and collectively referred to herein as non-analyte sensor 206).

In certain embodiments, a continuous analyte sensor 202 may comprise a sensor for detecting and/or measuring analyte(s). The continuous analyte sensor 202 may be a multi-analyte sensor configured to continuously measure two or more analytes or a single analyte sensor configured to continuously measure a single analyte as a non-invasive device, a subcutaneous device, a transcutaneous device, a transdermal device, and/or an intravascular device. In certain embodiments, the continuous analyte sensor 202 may be configured to continuously measure analyte levels of a user using one or more measurement techniques, such as enzymatic, chemical, physical, electrochemical, spectrophotometric, polarimetric, calorimetric, iontophoretic, radiometric, immunochemical, and the like. In certain aspects the continuous analyte sensor 202 provides a data stream indicative of the concentration of one or more analytes in the user. The data stream may include raw data signals, which are then converted into a calibrated and/or filtered data stream used to provide estimated analyte value(s) to the user.

In certain embodiments, continuous analyte sensor 202 may be a multi-analyte sensor, configured to continuously measure multiple analytes in a user's body. For example, in certain embodiments, the continuous multi-analyte sensor 202 may be a single multi-analyte sensor configured to measure two or more of glucose, insulin, lactate, ketones, pyruvate, and potassium in the user's body.

In certain embodiments, one or more multi-analyte sensors may be used in combination with one or more single analyte sensors. As an illustrative example, a multi-analyte sensor may be configured to continuously measure potassium and glucose and may, in some cases, be used in combination with an analyte sensor configured to measure only lactate levels. Information from each of the multi-analyte sensor(s) and single analyte sensor(s) may be combined to provide diabetes decision support using methods described herein.

The continuous analyte sensor 202 may be implemented as a continuous glucose monitor (CGM). Some examples of a continuous glucose monitor include a glucose monitoring sensor. In some embodiments, glucose monitoring sensor is an implantable sensor, such as described with reference to U.S. Pat. No. 6,001,067 and U.S. Patent Publication No. US-2011-0027127-A1. In some embodiments, the glucose monitoring sensor is a transcutaneous sensor, such as described with reference to U.S. Patent Publication No. US-2006- 0020187-A1. In yet other embodiments, the glucose monitoring sensor is a dual electrode analyte sensor, such as described with reference to U.S. Patent Publication No. US-2009-0137887-A1. In still other embodiments, the glucose monitoring sensor is configured to be implanted in a host vessel or extracorporeally, such as the sensor described in U.S. Patent Publication No. US-2007-0027385- A1. These patents and publications are incorporated herein by reference in their entirety.

As used herein, the term "continuous" may mean fully continuous, semi-continuous, periodic, etc. Such continuous monitoring of analytes is advantageous in diagnosing and staging a disease given the continuous measurements provide continuously up to date measurements as well as information on the trend and rate of analyte change over a continuous period. Such information may be used to make more informed decisions in the assessment of glucose homeostasis and treatment of diabetes.

In certain embodiments, sensor electronics module 204 includes electronic circuitry associated with measuring and processing the continuous analyte sensor data, including prospective algorithms associated with processing and calibration of the sensor data. Sensor electronics module 204 can be physically connected to continuous analyte sensor(s) 202 and can be integral with (non-releasably attached to) or releasably attachable to continuous analyte sensor(s) 202. Sensor electronics module 204 may include hardware, firmware, and/or software that enables measurement of levels of analyte(s) via a continuous analyte sensor(s) 202. For example, sensor electronics module 204 can include a potentiostat, a power source for providing power to the sensor, other components useful for signal processing and data storage, and a telemetry module for transmitting data from the sensor electronics module to one or more display devices. Electronics can be affixed to a printed circuit board (PCB), or the like, and can take a variety of forms. For example, the electronics can take the form of an integrated circuit (IC), such as an Application-Specific Integrated Circuit (ASIC), a microcontroller, and/or a processor.

Display devices 210, 220, 230, and/or 240 are configured for displaying displayable sensor data, including analyte data, which may be transmitted by sensor electronics module 204. Each of display devices 210, 220, 230, or 240 can include a display such as a touchscreen display 212, 222, 232, /or 242 for displaying sensor data to a user and/or receiving inputs from the user. For example, a graphical user interface (GUI) may be presented to the user for such purposes. In some embodiments, the display devices may include other types of user interfaces such as a voice user interface instead of, or in addition to, a touchscreen display for communicating sensor data to the user of the display device and/or receiving user inputs. Display devices 210, 220, 230, and 240 may be examples of display device 107 illustrated in FIG. 1 used to display sensor data to a user of FIG. 1 and/or receive input from the user.

In some embodiments, one, some, or all of the display devices are configured to display or otherwise communicate the sensor data as it is communicated from the sensor electronics module (e.g., in a data package that is transmitted to respective display devices), without any additional prospective processing required for calibration and real-time display of the sensor data.

The plurality of display devices may include a custom display device specially designed for displaying certain types of displayable sensor data associated with analyte data received from sensor electronics module. In certain embodiments, the plurality of display devices may be configured for providing alerts/alarms based on the displayable sensor data. Display device 210 is an example of such a custom device. In some embodiments, one of the plurality of display devices is a smartphone, such as display device 220 which represents a mobile phone, using a commercially available operating system (OS), and configured to display a graphical representation of the continuous sensor data (e.g., including current and historic data). Other display devices can include other hand-held devices, such as display device 230 which represents a tablet, display device 240 which represents a smart watch, medical device 208 (e.g., an insulin delivery device or a blood glucose meter), and/or a desktop or laptop computer (not shown).

Because different display devices provide different user interfaces, content of the data packages (e.g., amount, format, and/or type of data to be displayed, alarms, and the like) can be customized (e.g., programmed differently by the manufacture and/or by an end user) for each particular display device. Accordingly, in certain embodiments, a plurality of different display devices can be in direct wireless communication with a sensor electronics module (e.g., such as an on-skin sensor electronics module 204 that is physically connected to continuous analyte sensor(s) 202) during a sensor session to enable a plurality of different types and/or levels of display and/or functionality associated with the displayable sensor data.

In certain embodiments, one or more of the display devices may each have a user interface that may include a variety of interfaces, such a liquid crystal display (LCD) for presenting a UI features, a vibrator, an audio transducer (e.g., speaker), a backlight (not shown), and/or the like. The components that comprise such a user interface may provide controls to interact with the user (e.g., the host). One or more UI features may allow, for example, toggle, menu selection, option selection, status selection, yes/no response to on-screen questions, a "turn off" function (e.g., for an alarm), an "acknowledged" function (e.g., for an alarm), a reset, and/or the like. The UI features may also provide the user with, for example, visual data output. The audio transducer (e.g., speaker) may provide audible signals in response to triggering of certain alerts, such as present and/or predicted conditions. In some example implementations, audible signals may be differentiated by tone, volume, duty cycle, pattern, duration, and/or the like. In some example implementations, the audible signal may be configured to be silenced (e.g., acknowledged or turned off) by pressing one or more buttons.

As mentioned, sensor electronics module 204 may be in communication with a medical device 208. Medical device 208 may be a passive device in some example embodiments of the disclosure. For example, medical device 208 may be an insulin pump for administering insulin to a user. For a variety of reasons, it may be desirable for such an insulin pump to receive and track glucose, potassium, lactate, insulin, ketone, and/or pyruvate values transmitted from continuous analyte monitoring system 104, where continuous analyte sensor 202 is configured to measure glucose, insulin, potassium, lactate, ketone and/or pyruvate.

Further, as mentioned, sensor electronics module 204 may also be in communication with other non-analyte sensors 206. Non-analyte sensors 206 may include, but are not limited to, an altimeter sensor, an accelerometer sensor, a temperature sensor, a respiration rate sensor, a sweat sensor, etc. Non-analyte sensors 206 may also include monitors such as heart rate monitors, ECG monitors, blood pressure monitors, pulse oximeters, caloric intake, and medicament delivery devices. One or more of these non-analyte sensors 206 may provide data to decision support engine 114 described further below. In some aspects, a user may manually provide some of the data for processing by training server system 140 and/or decision support engine 114 of FIG. 1.

In certain embodiments, the non-analyte sensors 206 may be combined in any other configuration, such as, for example, combined with one or more continuous analyte sensors 202. As an illustrative example, a non-analyte sensor, e.g., a heart rate sensor, may be combined with a continuous analyte sensor 202 configured to measure glucose to form a glucose/heart rate sensor used to transmit sensor data to sensor electronics module 204 using common communication circuitry. As another illustrative example, a non-analyte sensor, e.g., a heart rate sensor and/or an ECG sensor, may be combined with a multi-analyte sensor 202 configured to measure glucose and potassium to form glucose/potassium/heart rate sensor used to transmit sensor data to the sensor electronics module 204 using common communication circuitry.

In certain embodiments, a wireless access point (WAP) may be used to couple one or more of continuous analyte monitoring system 104, the plurality of display devices, medical device(s) 208, and/or non-analyte sensor(s) 206 to one another. For example, WAP 138 may provide Wi-Fi and/or cellular connectivity among these devices. Near Field Communication (NFC) and/or Bluetooth may also be used among devices depicted in diagram 200 of FIG. 2.

FIG. 3 illustrates example inputs that are used by the decision support system of FIG. 1, according to some embodiments disclosed herein. In particular, FIG. 3 provides a more detailed illustration of example inputs introduced in FIG. 1. FIG. 3 illustrates example inputs 128 on the left, application 106 and decision support engine 114 including prediction module 116 in the middle, and an alert 130 on the right. In certain embodiments, the alert 130 corresponds to an event predicted using the prediction module 116 and may describe the event.

Application 106 obtains inputs 128 through one or more channels (e.g., manual user input, sensors/monitors, other applications executing on display device 107, EMRs, etc.). As mentioned previously, in certain embodiments, inputs 128 may be processed by the prediction module 116 and/or decision support engine 114 to output decision support outputs (e.g., alerts 130). As described above, alerts 130 are only one example of the type of decision support output that may be provided to the user. For example, in certain embodiments, inputs 128 may be used by decision support engine 114 to provide additional or alternative decision support outputs to the user, such as decision support recommendations, signals to an insulin delivery device (e.g., an insulin pump or pen).

The inputs 128 may include glucose measurements at a given time t(k) (g(k)), meal intake information at a given time t(k) (CHO(k)), and an amount of exogenous insulin information administered at a given time t(k) (I(k)). Note that the time at which glucose is measured, meals are consumed, and exogenous insulin are administered may be different.

The glucose measurements g(k) may be measured by and received from at least a continuous glucose sensor (or multi-analyte sensor configured to measure at least glucose) that is a part of continuous analyte monitoring system 104. Meal intake information CHO(k) may include information about one or more of meals, snacks, and/or beverages, such as one or more of the size, content (milligrams (mg) of potassium, glucose, lactate, carbohydrate, fat, protein, etc.), sequence of consumption, and time of consumption. In certain embodiments, meal intake information CHO(k) may be provided by a user through manual entry, by providing a photograph through an application that is configured to recognize food types and quantities (e.g., potassium and glucose/carbohydrate content of foods), and/or by scanning a bar code or menu. In various examples, meal size may be manually entered as one or more of calories, quantity (e.g., "three cookies"), menu items (e.g., "Royale with Cheese"), and/or food exchanges (e.g., 1 fruit, 1 dairy). In some examples, meal intake information CHO(k) may be received via a convenient user interface provided by application 106.

In certain embodiments, meal intake information CHO(k) entered by a user may relate to glucose consumed by the user. Glucose for consumption may include any natural or designed food or beverage that contains glucose, dextrose or carbohydrate, such as glucose tablet, a banana, or bread, for example.

The exogenous insulin information I(k) may include information relating to a user's insulin delivery. In particular, input related to the user's insulin delivery may be received, via a wireless connection on a smart insulin pen, via user input, and/or from an insulin pump. Insulin delivery information may include one or more of insulin volume, time of delivery, etc. Other parameters, such as insulin action time, insulin activity rate or duration of insulin action, may also be received as inputs.

In certain embodiments, time may also be provided as an input, such as time of day or time from a real-time clock. For example, in certain embodiments, glucose measurements g(k), meal intake information (CHO(k)), and exogenous insulin information I(k) may be timestamped to indicate a date and time when the information was received for the user.

User input of any of the above-mentioned inputs 128 may be through a user interface of display device 107 of FIG. 1. As described above, in certain embodiments, the prediction module 116 determines whether or not to provide a decision support output and the content of such output (e.g., whether or not to generate an alert 130 and the content of an alert 130) based on inputs 128.

*Example Methods and Systems for Providing Predictive Decision Support Outputs Using a Prediction Module*

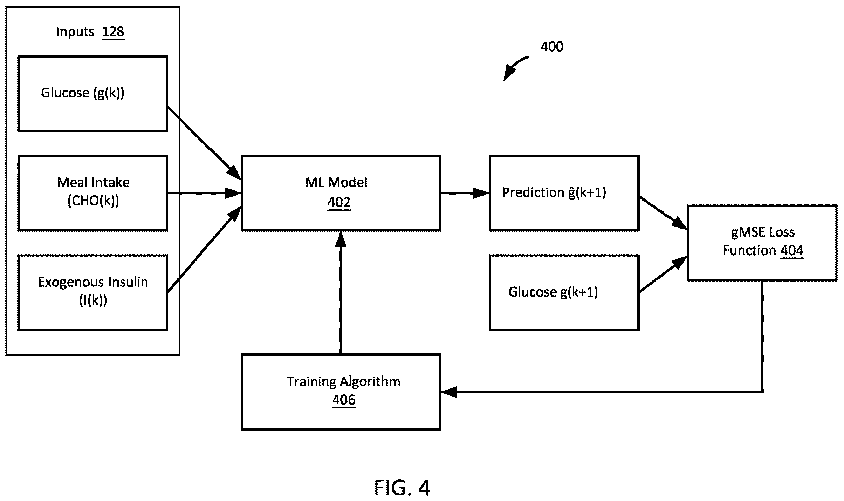

FIG. 4 illustrates an example workflow 400 for training a machine learning model 402 configured to predict future glucose measurements based on the inputs 128. The machine learning model 402 may be trained using the inputs 128 for an individual user or a population of users. The machine learning model 402 may be used by the prediction module 116 to obtain predicted future glucose measurements in order to determine whether or not to generate a decision support output (e.g., alert 130) as well as the content of such outputs (e.g., whether or not to generate an alert 130 and the content of the alert 130 as described in greater detail below). Training of the machine learning model 402 may be performed by the training server system 140 or by a software module within the prediction module 116 itself. The workflow 400 may be performed continuously or periodically in order to update the machine learning model 402 over time.

For a given time t(k), the inputs 128 for that time t(k), or a window preceding the time t(k), are processed using the machine learning model 402 to obtain a predicted glucose measurement ĝ(k+1) for a subsequent time t(k+1). As used herein "t(k)" or "t(k+n)," where n is any integer, identifies a time value in a regular or irregularly spaced series of time values with respect to which the workflow 400 is performed, such as every 5 minutes, every 15 minutes, or other interval. As noted above, the inputs 128 may not be received simultaneously such that, for a given time t(k), the value of an input 128 (g(k), CHO(k), or I(k)) may be obtained by interpolation, extrapolation, curve fitting, or other approaches to infer a value of the input 128 at a time t(k) for which no value is available for the input 128.

The predicted glucose measurement ĝ(k+1) and an actual measured glucose value g(k+1) for time t(k+1) are then processed according to a loss function 404. The loss function may be any loss function known in the art, such as a mean squared error (MSE). The loss function may also be the glucose-specific mean squared error (gMSE) described in [32] and summarized below. The value ĝ(k+1) may be obtained by interpolation, extrapolation, curve fitting or other approaches based on glucose measurements obtained at a time other than that corresponding to g(k+1). The output of the loss function 404 is then input to a training algorithm 406. The training algorithm 406 then updates one or more parameters of the machine learning model 400 according to the output of the loss function 404 such that the machine learning model 402 is trained to predict glucose measurements based on past values of the inputs 128.

The machine learning model 402 may be a linear regression-based model or other type of machine learning model such as non-linear machine learning models. The machine learning model 402 is a multi-input, single output (MISO) model. MISO models show a large number of degrees of freedom that can be selected for implementing the machine learning model 402, for instance: the model class (autoregressive with exogenous input (ARX); autoregressive moving average with exogenous input (ARMAX); autoregressive integrated moving average with exogenous input (ARIMAX); output-error (OE); Box-Jenkins, (BJ)) and the model complexity (number of parameters to be estimated). The test results summarized below were obtained using ARIMAX models with Bayesian information criterion (BIC) used as the method to select the model orders (see [24], [29], [30]).

To estimate model parameters of the machine learning model 402, the prediction error method (PEM) may be used to minimize the one-step ahead prediction error. In particular, let the model parameters be designated as $\theta$ and let the loss function 404 be implemented as an MSE cost function. Estimated model parameters $\hat{\theta}$ may therefore be calculated according to equation (1) below, where k is an index corresponding to inputs 128 for a given time value in the series of time values and $\hat{g}(k + 1)$ is the predicted glucose measurement for the next time value in the series of time values, and MSE is defined according to equation (2) below, where N is the number of available data samples.

$$\hat{\theta} = \underset{\theta}{\operatorname{argmin}} \, MSE(g(k), \hat{g}(k + 1|k, \theta)) \qquad (1)$$

$$MSE(g(k), \hat{g}(k + 1|k, \theta)) = \frac{1}{N} \sum_{1}^{N} (g(k) - \hat{g}(k + 1|k, \theta))^2 \qquad (2)$$

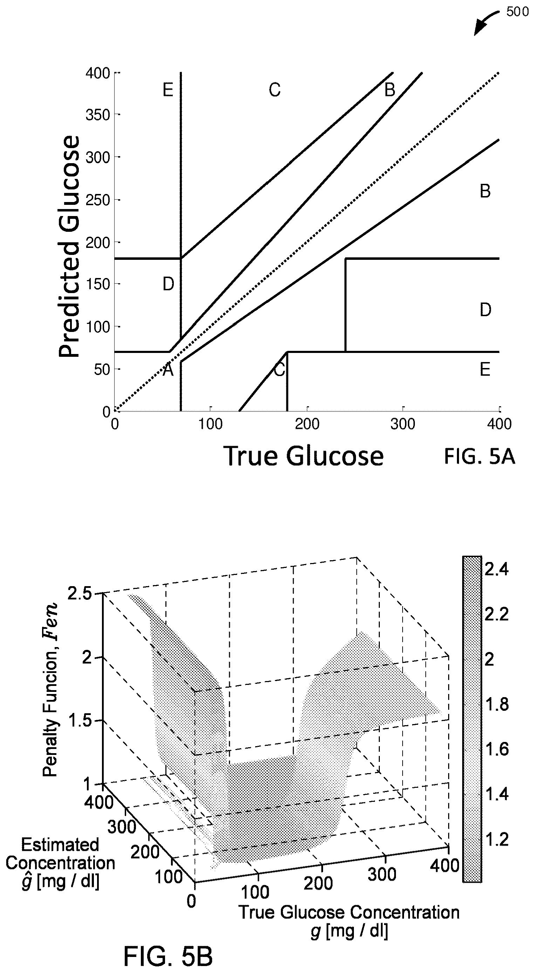

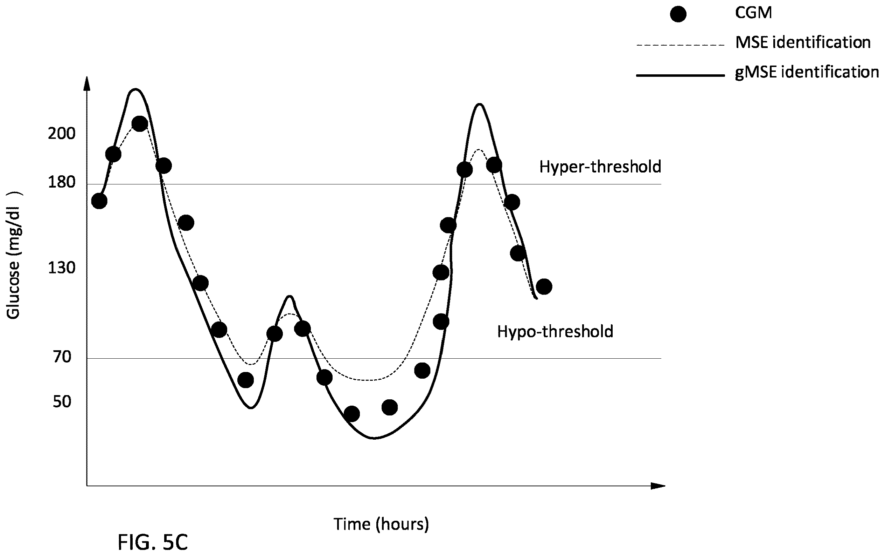

Referring to FIGS. 5A, 5B, and 5C, in an improved approach the estimated model parameters $\hat{\theta}$ may be

calculated according to equation (3) using the gMSE described in [32].

$$\hat{\theta} = \underset{\theta}{\operatorname{argmin}} gMSE(g(k), \hat{g}(k + 1|k, \theta)) \qquad (3)$$

The gMSE metric is inspired by the Clarke error grid (CEG) [36] shown in FIG. 5A and accounts for the clinical impact of the prediction error. The gMSE metric is obtained by increasing MSE values according to a penalty function in the case of overestimation of hypoglycemia and underestimation of hyperglycemia. For example, the gMSE value may be obtained by increasing the MSE value by up to 250% in case of glucose over-estimation during hypoglycemia and up to 200% in case of glucose under-estimation in hyperglycemia. By doing so, over-estimation in hypoglycemia is penalized more than under-estimation in the same region, since the first is clinically more dangerous: over-estimation in hypoglycemia could prevent the detection of the episode or induce an optimizing evaluation of its severity, leading to inadequate treatment. A symmetric reasoning holds for the case of hyperglycemia but, since hypoglycemia is deemed more dangerous than hyperglycemia, in the first case, MSE is increased more (e.g., up to 250%) than in the second case (e.g., only up to 200%).

The MSE increase is done so that gMSE retains two fundamental mathematical properties of the original metric, smoothness and convexity, as these properties simplify the optimization involved in the parameters estimation. In particular, the penalty function may include a sigmoid function of $g(k + 1)$ and $\hat{g}(k + 1)$ as described in [32] to achieve a smooth surface as shown in FIG. 5B. For example, zones E and D in the Clarke error grid of FIG. 5A indicate dangerous failure to accurately detect glucose levels. Accordingly, as shown in Fig. 5B, the penalty function may increase steeply with a finite slope at the boundaries of zones E and D.

Referring to FIG. 5C, the machine learning model 402 trained using the gMSE as the loss function 404 will output predicted values that are likely to be more accurate in the vicinity of and above the hyper-glycemic threshold (e.g., 180 mg/dl) relative to a model trained using only the MSE, since errors in this region were penalized more during training. Overestimation errors are favored as compared to underestimation error in this region (vicinity of and above the hyper-glycemic threshold), since overestimation errors are less likely to cause clinically impactful damage. Likewise, the machine learning model 402 trained using the gMSE as the loss function n404 will output predicted values that are likely to be more accurate in the vicinity of the hypo-glycemic threshold (e.g., 70 mg/dl) with respect to MSE, since errors in this region were penalized more during training Underestimation errors are favored as compared to overestimation errors in this region (vicinity of the hypo-glycemic threshold), since they are less likely to cause clinically impactful damage.

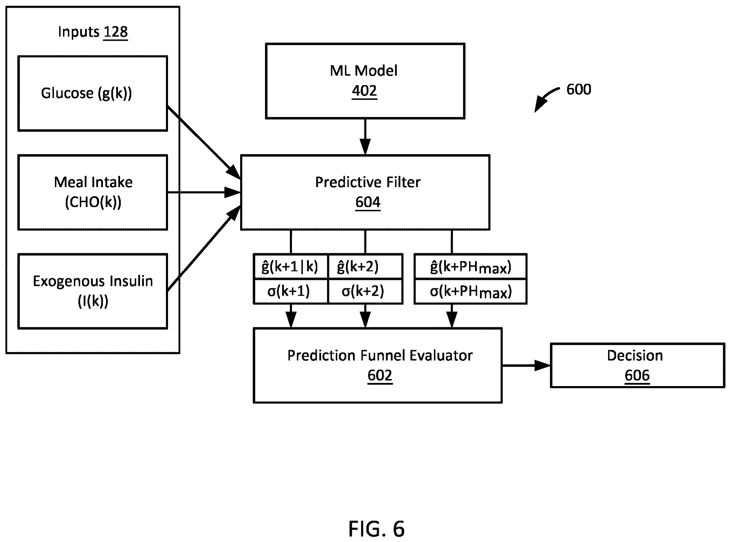

FIG. 6 illustrates a workflow 600 including a prediction funnel evaluator 602 for determining the appropriateness of generating a decision support output, such as an alert 130, based on glucose values predicted using the machine learning model 402. Although the workflow 600 is shown and described with reference to a machine learning model 402 trained using the gMSE, other machine learning models trained using the MSE or other loss functions may be used in place of the machine learning model 402 while still achieving at least some of the benefit of the prediction funnel evaluator 602.

Several prior approaches for generating alerts using predicted glucose values proposed in literature focus on a single prediction and seldom account for prediction accuracy in detecting the crossing of the hypoglycemic threshold [14], [15]. In contrast, the prediction funnel implemented by the workflow 600 simultaneously considers multiple predictions at different prediction horizons while also accounting for the uncertainty of the predictions.

In particular, the machine learning model 402 is used to build a predictive filter 604, such as a Kalman predictor using the standard procedure described in [26]. The predictive filter 604 receives the inputs 128 for a time value t(k) and outputs predicted glucose measurements $\hat{g}(k + 1|k)$ to $\hat{g}(k + PH_{max}|k)$ from the machine learning model 402, where $PH_{max}$ is the maximum prediction horizon. The value of $PH_{max}$ is a configurable value and may have any value greater than 1. For example, assuming time values at 5 minute intervals, a one hour maximum prediction horizon may be achieved with $PH_{max}$ = 12. The predictive filter 604 further outputs, for each prediction, a corresponding uncertainty, such as variances $\sigma^2(k + 1|k)$ to $\sigma^2(k + PH_{max}|k)$.

The predictions $\hat{g}(k + 1|k)$ to $\hat{g}(k + PH_{max}|k)$ and corresponding uncertainties, e.g., variances $\sigma^2(k + 1|k)$ to $\sigma^2(k + PH_{max}|k)$, are then input to the prediction funnel evaluator 602, which outputs a decision 606. The decision 606 may be any of a hypoglycemic alert, hyperglycemic alert, or a decision not to generate any alert. Decision 606 may instead or additionally also include other types of decision support outputs. In one example, if a hypoglycemic alert is appropriate, a decision support recommendation to consume glucose may also be provided.

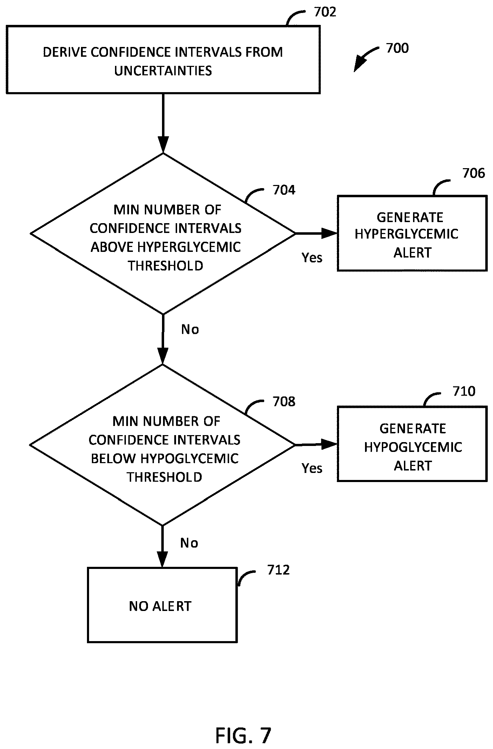

FIG. 7 illustrates an example method 700 implemented by the prediction funnel evaluator 602. The method 700 may be executed for each time value t(k) with respect to predictions $\hat{g}(k + 1|k)$ to $\hat{g}(k + PH_{max}|k)$ and corresponding uncertainties, e.g., variances $\sigma^2(k + 1|k)$ to $\sigma^2(k + PH_{max}|k)$, for time value t(k).

The method 700 may include deriving, at step 702, confidence intervals from the uncertainties received from the predictive filter 604. For example, for a given prediction $\hat{g}(k + i|k)$ and corresponding variance $\sigma^2(k + i|k)$, the confidence interval may be calculated as $\hat{g}(k + i|k) \pm m\sigma(k + i|k)$. The value of $m$ is a configurable parameter that is used to adjust the probability $\alpha(m)$ that the confidence interval is entirely above the hyperglycemic threshold or entirely below the hypoglycemic threshold. Increasing $m$ decreases $\alpha(m)$ whereas decreasing $m$ increases $\alpha(m)$. The set of confidence intervals $\hat{g}(k + i|k) \pm m\sigma(k + i|k)$, i = 1 to $PH_{max}$, is referred to herein as "the prediction funnel."

The method 700 includes evaluating, at step 704, whether a number of the confidence intervals entirely above the hyperglycemic threshold (e.g., 180 mg/dl) is greater than or equal to a minimum number $N_{pred}$. $N_{pred}$ is a tunable value that may be any number from 1 to $PH_{max}$. Concerning hypoglycemia alarms, experiments conducted by the inventors have found that using a value of 1 for $N_{pred}$ provides suitable results, though the benefits of the prediction funnel evaluator 602 may be achieved with other values as well. If the condition of step 704 is met, then the method 700 includes predicting a hyperglycemic event and/or invoking, at step 706, generation of a hyperglycemic alert 130.

The method 700 may include evaluating, at step 708, whether a number of the confidence intervals entirely below the hypoglycemic threshold (e.g., 70 mg/dl) is greater than or equal to $N_{pred}$. The value of $N_{pred}$ may be the same for the evaluations of step 704 and 708 or may be different. If the condition of step 708 is met, then the method 700 includes predicting a hypoglycemic event and/or invoking, at step 710, generation of a hyperglycemic alert 130. If neither of the conditions of steps 704 and 708 are met, then the method ends at step 712 without predicting a hypoglycemic or a hyperglycemic event and invoking generation of an alert 130.

Decision support engine 114 may generate a hypo- or hyperglycemic alert 130 by causing the display device 107 to provide a human-perceptible alert, such as a visible message, audible alert, or palpable alert (e.g., with a haptic device). Decision support engine 114 may instead or additionally generate an instruction to another device to generate a visible, audible, or palpable alert, such as a fitness tracker, smart watch, or other wearable computing device.

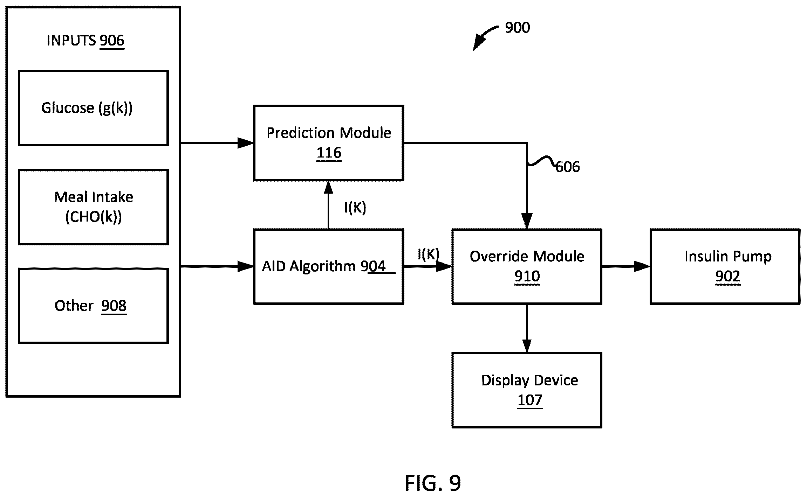

If a hypoglycemic or a hyperglycemic event is predicted, in certain embodiments, additional or alternative decision support output may be provided to the patient. For example, the decision support output may include a decision support recommendation for the patient to engage in exercise, consume carbohydrates, administer insulin, etc. In certain embodiments, the decision support output may further include a signal to an insulin pump to alter the amount of insulin that is being or to be administered to a user, as described below with respect to Fig. 9.

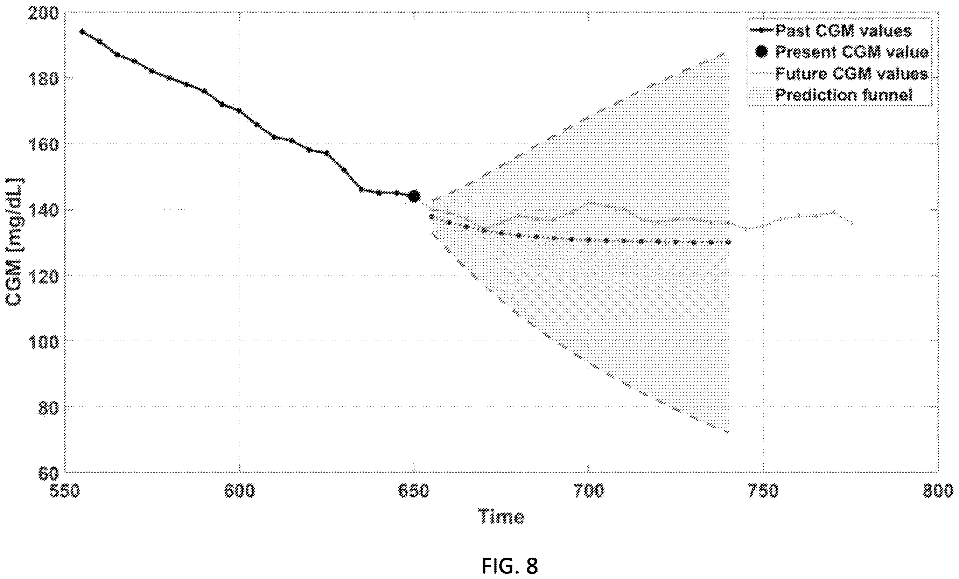

FIG. 8 is a plot visualizing a prediction funnel. As is apparent in Fig. 8, the funnel increases in height with temporal distance from the present glucose measurement, i.e., g(k). This increase in height corresponds to the increasing size of the confidence intervals of predicted glucose measurements with a temporal distance from g(k). Accordingly, the probability that a confidence interval will be completely above the hyperglycemic threshold or completely below the hypoglycemic threshold decreases with the temporal distance to the future time t(k+i) corresponding to a given prediction g(k+i|k). The prediction funnel, therefore, has the benefit of evaluating multiple prediction horizons while at the same time taking into account that uncertainty increases and the prediction horizon increases.

Various modifications to the prediction funnel evaluator 602 may be made while still achieving at least some of the benefits described above.

First, various types and numbers of machine learning models may be used in place of the machine learning model 402 described above. For example, a non-linear physiological model may be used in place of the ARIMAX machine learning model. For some alternative machine learning model types, it may not be feasible to use a predictive filter 604 to obtain predictions more than one time step ahead. Instead, multiple machine learning models may be trained, each with a different prediction horizon and providing a corresponding confidence interval or metric of uncertainty that may be used to obtain a confidence interval.

Some types of alternative machine learning models do not inherently provide a confidence interval or an uncertainty that may be used to derive a confidence interval. Therefore, a confidence interval may be computed independently of the machine learning model or models by estimating prediction error variance for a training set at each prediction horizon and using the estimated prediction error variance for each prediction horizon to obtain the confidence interval at each prediction horizon.

Some example alternatives to the use of an ARIMAX machine learning model 402 with a Kalman filter as the predictive filter 604 include at least (a) a non-linear physiological model of glucose-insulin dynamics or other non-linear black box model (e.g., deep neural network (DNN), convolution neural network (CNN), etc.) combined with (b) any of an extended Kalman filter, unscented Kalman filter, or particle filter.

In another alternative, an ensemble approach is used in which multiple models of any of the above-described types are fit to a training set and a distribution of the prediction errors for the training set is calculated for each of the multiple models. The distribution of prediction errors of each model may then be used to weight the prediction of each model to compute a new estimate for each model, e.g., via linear combination. The new estimates for the models may then be combined to obtain an ensemble prediction by selecting the new estimate with the lowest prediction error, averaging the new estimates, or another approach. The error variance of the ensemble prediction can be obtained from the training dataset or derived from the distribution of prediction errors for each model. Predictions at different prediction horizons and the error variance of the ensemble can then be used to build the prediction-funnel as described above.

Referring to FIG. 9, the illustrated workflow 900 may be used to control delivery of insulin and/or performance of other ameliorating actions in place of or in addition to generating a human-perceptible output in response to an alert 130.

In the workflow 900, exogenous insulin I(k) delivered by an insulin pump 902 is calculated by an automatic insulin delivery (AID) algorithm 904 based on the inputs 906, which may include the glucose measurements g(k), meal intake information CHO(k), and possibly other values 908 received from the user or from any of the sensors, such as any of the non-analyte sensors 206, described herein above. The AID algorithm 904 may be any AID algorithm known in the art.

Control signals from the AID algorithm 904 directing the insulin pump 902 to provide a particular rate of insulin delivery may be received by an override module 910. The override module 910 refers to a set of software instructions that may be provided as part of decision support engine 114. The override module 910 may either pass the control signals from the AID algorithm 904 to the insulin pump 902, completely suppress the control signals, or modify the control signals to change the rate of insulin delivery relative to that dictated by the AID algorithm 904. The override module 910 may take as an input the decision 606 output by the prediction funnel evaluator 602. Decision 606, as described above, may indicate whether a hypo- or hyperglycemic event has been predicted.

In a first implementation, in the event of a predicted hypoglycemic event, the override module 910 completely suppresses infusion of insulin by the insulin pump 902 until the decision 606 no longer indicates a hypoglycemic event.

In a second implementation, the override module 910 receives the prediction funnel, i.e., the confidence intervals for each glucose prediction from the predictive filter 604, and adjusts the amount of insulin infusion instructed by the AID algorithm 904 according to the prediction funnel. For example, the override module 910 may reduce insulin infusion by 50% if the prediction funnel is below 100 mg/dl, by 75% if the funnel is below 80 md/dl, and by 100% if the prediction funnel is below 70 mg/dl.

In a third implementation, the value of I(k) used to control infusion by the insulin pump 902 is selected using the workflow 600. For example, let $I(k_1)$ be the insulin infusion amount selected by the AID algorithm 904. The override module 910 may select a new insulin infusion amount $I(k_2)$ by generating a set of insulin amounts $I(k_1)+\delta_j$, j = 1 to P, where P is the number of insulin amounts and the values of $\delta_j$ are selected to provide a range of insulin infusion including $I(k_1)$. A plurality of prediction funnels may be obtained according to the workflow 600, each prediction funnel being calculated using g(k) and CHO(k) and one the values $I(k1)+\delta_j$. The value of $I(k_2)$ selected by the override module 910 to control insulin infusion by the insulin pump 902 may be one of the values $I(k_1)+\delta_j$ for which the corresponding prediction funnel was entirely above 70 mg/dl. If none of the prediction funnels is entirely above 70 mg/dl, the override module 910 may output an instruction on the display device 107 to consume a prescribed amount of carbohydrates, e.g., 10, 15, or 20 grams.

The second or third implementation may be replaced with or used in conjunction with providing a decision support recommendation for output by the display device 107. For example, in the event of a predicted hypoglycemic event, the override module 910 instructs the display device 107 to output decision support recommendation to consume a prescribed amount of carbohydrates, e.g., 15 grams. The prescribed amount of carbohydrates may be fixed or determined based on the prediction funnel, e.g., 10 grams if the prediction funnel is partially below 70 mg/dl but all above 50 mg/dl; 15 grams if the funnel is entirely below 70 mg/dl and partially below 50 mg/dl; and 20 grams if the funnel is entirely below 50 mg/dl.

The prescribed amount of carbohydrates may also be determined using the workflow 600. For example, a plurality of prediction funnels may be obtained according to the workflow 600, each prediction funnel being calculated using g(k) and I(k) and one a plurality of alternative values for CHO(k), such as a range of alternative values including the current value of CHO(k). The prescribed amount of carbohydrates selected by the override module 910 may be that which for which the corresponding prediction funnel was entirely above 70 mg/dl.

*Test Results*

Test results were obtained using the improved training of the prediction model described with respect to FIGS. 4 to 5C and the alarm-raising model described with respect to FIGS. 6 to 8. Testing was performed using data collected in a multicenter clinical trial (NCT02137512) and aimed to assess the long-term term use of an hybrid closed-loop insulin delivery system (Artificial Pancreas) developed at the University of Virginia [32]. The study and all experimental procedures were approved by local IRB/ethical committee. Fourteen (Type 1 diabetes) T1D individuals participated to the 5-month main phase testing 24/7 the use of the Artificial Pancreas system. Glucose data were recorded using a DexCom G4® sensor (DexCom, Inc., San Diego, CA, USA) with a sample time of 5 min, whereas insulin was infused with a Roche Accu-Check Spirit Combo® insulin pump (Roche Diabetes Care, Inc., Indianapolis, IN, USA). These two off-the-shelf medical devices used Bluetooth to communicate with the principal system component, the Diabetes Assistant (DiAs) [33], a platform based on an Android smartphone in charge of managing the data-flow, offering a friendly user-interface and hosting the control algorithm. Individuals were instructed to manually deliver a proper amount of insulin for all meals, by inserting in the system all carbohydrate consumption. Further details of the experiment are described in [32].

The test data was pre-processed to deal with experimental data of different natures (CGM (continuous glucose monitor), insulin information and carbohydrates intake), which poses some technical issues related to device synchronization, completeness of stored data and reliability of patient's provided information. Firstly, to fix synchronization problems, all signals were aligned to the same time grid equally sampled at $T_S$ = 5 min. Secondly, a portion of 14 consecutive days of data containing only incomplete data portions lasting less than 30 min, which is suitable for the purposes of this work, was selected and then split in training-set (first 7 days) and test-set (last 7 days). Lastly, in relation to missing information, incomplete data portions lasting less than 30 min were differently filled depending on whether the gap occurred in training or test data, as described in the following. According to this data preprocessing step, three subjects were excluded from the analysis because a lack of sufficient acceptable data. Finally, regarding the missing data, in the training-set a third-order spline interpolation was used to fill the remaining missing samples contained in the time series. Indeed, the training-set is entirely available during model training, and non-causal techniques can be used. Instead, on the test-set glucose prediction should be performed only in real-time. For this reason, only the past samples were used to close the gaps, using a zero-order hold that is applicable in real-time.

Dealing with experimental data of different natures (CGM, insulin information and carbohydrates intake) poses some technical issues related to device synchronization, completeness of stored data and reliability of patient's provided information. Firstly, to fix synchronization problems, all signals were aligned to the same time grid equally sampled at TS = 5 min. Secondly, a portion of 14 consecutive days of data containing only incomplete data portions lasting less than 30 min, which is suitable for the purposes of the experiment, was selected and then split in training-set (first 7 days) and test-set (last 7 days). Lastly, in relation to missing information, incomplete data portions lasting less than 30 min were differently filled depending on whether the gap occurred in training or test data, as described in the following. According to this data preprocessing step, three subjects were excluded from the analysis because enough acceptable data could not be found. Finally, regarding the missing data, in the training-set a third-order spline interpolation was used to fill the remaining missing samples contained in the time series. Indeed, the training-set is entirely available during model training, and non-causal techniques can be used. Instead, on the test-set glucose prediction may be performed only in real-time. For this reason, only the past samples were used to close the gaps, using a zero-order hold that is applicable in real-time.

Following the definition proposed in the consensus paper [39] by a group of experts in the field, an hypoglycemic episode (HE) has occurred at time t(k) if the BG is below 70 mg/dL for a period of at least 15 minutes. The episode continues until the BG remains above this threshold for at least 15 minutes. A true positive (TP) occurs if an HE occurred at time t(k) and an alarm is generated before the event, precisely in a window from 60 and 5 minutes before t(k). According to this definition, only the alarms which are relatively close to the HE are considered correct, while alarms too far apart in the past are not counted as TP. A false positive (FP) occurs if an alarm is raised at time t(k) but no hypoglycemia occurred in the following 60 minutes. A false negative occurs if an HE occurred at time t(k) but no alarms were generated in the previous 60 minutes. Late alarms are defined as alarms at time t(k) or up to 15 minutes after. These are not counted as TP as they were not timely. On the contrary, late alarms increase the count of FN. Nonetheless, late alarms cannot be considered as erroneous, so late alarms do not increase the FP count.

Once the events were labeled as TP, FP, and FN, the following metrics were used to evaluate the state-of-art and the proposed approaches:

$$Precison: P = 100 \cdot \frac{TP}{TP+FP} \qquad (4)$$

$$Recall: R = 100 \cdot \frac{TP}{TP+FN} \qquad (5)$$

$$F1\ Score: F1 = 2 \cdot \frac{P \cdot R}{P+R} \qquad (6)$$

Precision is the fraction of the correct alarms over the total number of raised alarms. Recall, also known as sensitivity, is the fraction of correctly detected hypoglycemic events over the total number of events. F1-score is the harmonic mean of the two previous metrics. Since the dataset is strongly unbalanced, the average number of FPs generated by the algorithm in one day (FP/day) was also evaluated. Finally, the results include the time gain (TG) of the hypoglycemic alarms as the time between when the alarm was raised by the algorithm and the start of the *HE*. According to the definition of *TP,* the maximum achievable *TG* is 60 min, while the lowest is 5 min. Due to the limited number of hypoglycemic events, the value of hypoglycemia prediction metrics was obtained by considering all the hypoglycemic events of different subjects as they belong to a unique time series. The results is then expressed as a single value for all the considered metrics but for *TG,* that is expressed as mean and standard deviation (SD) of the time gain of every detections.

Table 1 shows the hypoglycemia prediction performances of several prediction algorithms. The first row reports the baseline performance achieved by the state-of-art algorithm (single PH, MSE) using individualized models, identified minimizing MSE, and considering only one prediction horizon, PH = 30 min. The last row of the table reports the performance achieved by the prediction model proposed herein, which includes the various aspects of the model (e.g., the use of *gMSE* for model identification and the prediction-funnel-based strategy).

Table 1. Hypoglycemia prediction evaluation metrics of considered approaches.

| Alarm Strategy | PH (min) | Cost Function | P[%] | R[%] | F1[%] | FP/day | TG [min] mean (SD) |
|---|---|---|---|---|---|---|---|
| Single PH | 30 | MSE | 43 | 95 | 59 | 0.77 | 15(10) |
| | | gMSE | 44 | 100 | 61 | 0.77 | 15(10) |
| | 45 | MSE | 37 | 83 | 51 | 0.86 | 15(10) |
| | | gMSE | 45 | 98 | 61 | 0.73 | 15(10) |
| | 60 | MSE | 36 | 76 | 49 | 0.82 | 20(15) |
| | | gMSE | 42 | 90 | 58 | 0.75 | 15(15) |
| Prediction Funnel (tuning 1) | 5 to 60 | MSE | 51 | 91 | 65 | 0.59 | 15(15) |
| | | gMSE | 51 | 96 | 66 | 0.59 | 15(15) |
| Prediction Funnel (tuning 2) | 5 to 60 | MSE | 62 | 76 | 68 | 0.33 | 15(15) |
| | | gMSE | 65 | 88 | 75 | 0.29 | 15(10) |

To elucidate the contribution of each of the aspects of the prediction model to the final performance, Table 1 reports also the performance achieved with inclusion of one modification at the time *(gMSE* + single-PH and MSE + prediction-funnel). Moreover, different values of the hyper-parameters are investigated. Focusing first on the single-PH strategy, the impact of PH on the prediction performance of the state-of-art approach was investigated by evaluating three possible PHs: PH = 30, 45, and 60 minutes. The best results were achieved with the PH = 30 min, which is in fact commonly adopted in literature. In particular, the larger the PH, the higher the *TG,* but at the expenses of a worse *P, R,* and FP/day. For instance, comparing the state-of-art approach with PH = 30 min and with PH = 60 min we can see that *TG* increases from 15 to 20 minutes (mean values), but P falls from 43% to 36% and R from 95% to 76%, while FP/day increases from 0.77 to 0.82. The introduction of the use of the *gMSE* in place of the MSE, improves both the precision and the recall with respect to the state-of-art approach. The improvement holds true for all considered values of PH. Considering for instance PH = 30 min, with the use of *gMSE, P* increases from 43% to 44%, R from 95% to 100%, while FP/day and *TG* are almost the same.

The impact of the improved alarmed strategy (prediction-funnel-based instead of using a single-PH) was also investigated. In particular, two different approaches to the tuning of the parameter *m* were considered. In both cases, a patient-specific value of *m* was considered, but in the first approach m was set in order to get a similar recall as the one achieved by the state-of-art (MSE + single-PH, PH = 30 min). As a second alternative approach m was chosen in each patient to maximize the *F1* in the training-set of that patient. The first approach is presented in Table 1 as *tuning 1,* whereas the second is denoted *tuning 2.*

The improvement achieved by the prediction-funnel is clearly visible with *tuning 1,* which offers higher precision, higher *F1* and less FP/day with respect to the state-of-art: P increase from 43% to 51%, *F1* from 59% to 65%, and FP/day decreased from 0.77 to 0.59. This improvement is achieved while retaining similar recall (R from 95% to 91%). The performances of *tuning 2* are more difficult to interpret, since it selects a different trade-off between precision and recall. Specifically, it renounces some recall in favor of a better precision and less FP/day, leading to an overall improvement in the F1-score.

Finally, combining the use of gMSE and the prediction funnel outperforms the state-of-art. Once again, this is clearly visible with *tuning 1*, which achieves similar recall and *TG* of the state-of-art (above 95%) but with higher precision, F1-score, and less false positives: P from 43% to 51%, *F1* from 59% to 69%, and false positives-per-day decreased from 0.77 to 0.59. By adopting a slightly more conservative approach, proposed in *tuning* 2, precision and false positives can be further improved (P = 65%, FP/day= 0.29, i.e., about one every 3 days), at the expenses of a deterioration of the recall *(R =* 88%). This new trade-off offers a better F1-score that reaches 75%.

The foregoing description provides a new approach to predict hypoglycemic events that is based on an individualized linear black-box model, identified by using an *ad-hoc* cost function designed to account for the clinical impact of a prediction error, and on an improved alarm strategy that considers the entire prediction-funnel. The results show that models identified via *gMSE* minimization provide better hypoglycemia prediction performances than models based on MSE. Furthermore, results show that the new alarm-raising model, based on the prediction-funnel, improve hypoglycemia detection, thanks to the possibility of exploiting multiple PHs. The adoption of both the proposed improvements grants the best performance.

It is important to note that, the definition of hypo-glycemic episode *(HE)* used to evaluate the prediction performances was formulated by a panel of international experts in the consensus paper [39]: an *HE* occurs if the BG is below 70 mg/dL for a period of at least 15 minutes. The same episode continues until the BG remains above this threshold for at least 15 minutes. According to this definition, when the BG falls below the hypoglycemic threshold only for one or two time samples, there is no hypoglycemic episode. If an alarm is raised in these cases (hereinafter "quasi-hypoglycemic episodes" *(qHE))*, the alarm will count as a *FP.*

Since a *FP* error caused by a *qHE* could be considered less clinically relevant that other FPs, it is of interest to investigate how many false positives are of this kind. For the state-of-art method (MSE + single-PH approach, PH = 30 min), 26% of the recorded FPs were associated to *qHE,* while this percentage raises to 34% with the newly proposed algorithm *(gMSE* + prediction-funnel), further supporting the superiority of the proposed algorithm. Discarding these events from the *FP* count, as frequently done in literature, would reduce the FP/day from 0.77 to 0.62 and increase the precision from 43% to 54% for the state-of-art approach while, for the proposed approach, would decrease FP/day from 0.29 to 0.21 and increase P form 65% to 73%.

Another consequence of the *HE* definition adopted, is that even a non-predictive hypoglycemia detector, simply based on the BG trace crossing the 70 mg/dL threshold, may raise false positive alarms (FP/day = 0.2). According to our definition of *TP,* the BG produces only late alarms (events will be detected exactly whenever they started, with *TG = 0* min). As a consequence, *TP* count is bound to be 0, and both recall and precision are necessarily 0, (i.e., P=R=0).

The test results show the benefit of including both model training using the gMSE and the use of the prediction funnel to determine the appropriateness of generating an alert when performing individualized hypoglycemia prediction. The test results show improvement with respect to prior contributions in this field [10], [14], [16], [17], [19], [20], [22]-[24]. These prior contributions should be interpreted with caution: a different definition of the events might significantly impact the final metrics (FP/day, P, R, F1, TG), as discussed above with respect to *qHE,* where a seemingly minor modification in the definition of *HE* has non negligible impact on FP/day and precision. Moreover, different validation dataset might be collected in very different conditions (highly controlled clinical trials vs. real-life) introducing a further confounding factor.

With this caveat in mind, the results obtained are comparable with most of other (both linear and non-linear regression-based) literature studies. Authors in [10], [19], [20] reached a recall, respectively, of about 93%, 93%, and 86%, comparable or slightly superior to the recall of the approach described herein, with R = 88%, at the expense of lower precision: about 24%, 38%, and 62%, while the approach described herein achieved P = 65%. Similarly, [14], [24] achieved the remarkable recall of *R* = 100%, but at the expense of a very high number of *FPs* (more than 1 *FPs* per day). Authors in [16], [17], [23] showed a similar recall to the one obtained using the approach described herein (89%, 94%, and 94%) with a better precision (78%, 90%, and 77%). However, the authors adopted a more permissive *HE* definition. For instance, [23] considered as *TPs* also alarms raised after the BG crossed the hypoglycemic threshold, whereas we consider them as *FNs.* In [16], performance was assessed using controlled inpatient data. Authors in [22] showed a slightly inferior recall (86%) and did not report any metrics related to false alarms.

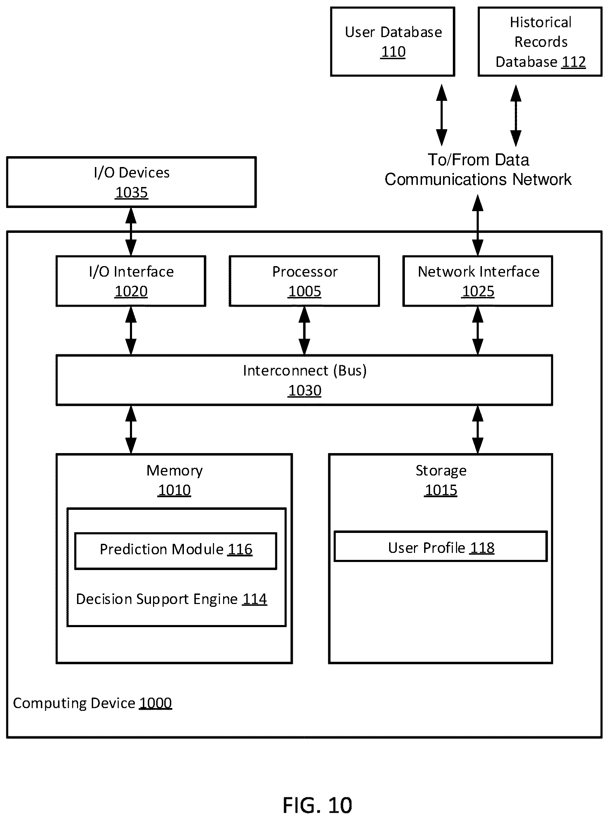

FIG. 10 is a block diagram depicting a computing device 1000 configured for predicting hypo- and hyperglycemic events, according to certain embodiments disclosed herein. Although depicted as a single physical device, in embodiments, computing device 1000 may be implemented using virtual device(s), and/or across a number of devices, such as in a cloud environment. As illustrated, computing device 1000 includes a processor 1005, memory 1010, storage 1015, a network interface 1025, and one or more I/O interfaces 1020. In the illustrated embodiment, processor 1005 retrieves and executes programming instructions stored in memory 1010, as well as stores and retrieves application data residing in storage 1015. Processor 1005 is generally representative of a single CPU and/or GPU, multiple CPUs and/or GPUs, a single CPU and/or GPU having multiple processing cores, and the like. Memory 1010 is generally included to be representative of a random access memory (RAM). Storage 1015 may be any combination of disk drives, flash-based storage devices, and the like, and may include fixed and/or removable storage devices, such as fixed disk drives, removable memory cards, caches, optical storage, network attached storage (NAS), or storage area networks (SAN).

In some embodiments, input and output (I/O) devices 1035 (such as keyboards, monitors, etc.) can be connected via the I/O interface(s) 1020. Further, via network interface 1025, computing device 1000 can be communicatively coupled with one or more other devices and components, such as user database 110 and/or historical records database 112. In certain embodiments, computing device 1000 is communicatively coupled with other devices via a network, which may include the Internet, local network(s), and the like. The network may include wired connections, wireless connections, or a combination of wired and wireless connections. As illustrated, processor 1005, memory 1010, storage 1015, network interface(s) 1025, and I/O interface(s) 1020 are communicatively coupled by one or more interconnects 1030. In certain embodiments, computing device 1000 is representative of display device 107 associated with the user. In certain embodiments, as discussed above, display device 107 can include the user's laptop, computer, smartphone, and the like. In another embodiment, computing device 1000 is a server executing in a cloud environment.

In the illustrated embodiment, storage 1015 includes user profile 118. Memory 1010 includes decision support engine 114, which itself includes the prediction module 116. Decision support engine 114 is executed by computing device 1000 to perform operations in workflow 400 of FIG. 4, operations the workflow 600 of FIG. 6, the method 700 of Fig. 7, and/or operations of the workflow 900 in FIG. 9.

*Additional Considerations*

The methods disclosed herein comprise one or more steps or actions for achieving the methods. The method steps and/or actions may be interchanged with one another without departing from the scope of the claims. In other words, unless a specific order of steps or actions is specified, the order and/or use of specific steps and/or actions may be modified without departing from the scope of the claims.

As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiples of the same element (e.g., a-a, a-a-a, a-a-b, a-a-c, a-b-b, acc, b-b, b-b-b, b-b-c, c-c, and c-c-c or any other ordering of a, b, and c).

The previous description is provided to enable any person skilled in the art to practice the various aspects described herein. Various modifications to these aspects will be readily apparent to those skilled in the art, and the generic principles defined herein may be applied to other aspects. Thus, the claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more. All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112(f) unless the element is expressly recited using the phrase "means for" or, in the case of a method claim, the element is recited using the phrase "step for."

While various examples of the invention have been described above, it should be understood that they have been presented by way of example only, and not by way of limitation. Likewise, the various diagrams may depict an example architectural or other configuration for the disclosure, which is done to aid in understanding the features and functionality that can be included in the disclosure. The disclosure is not restricted to the illustrated example architectures or configurations, but can be implemented using a variety of alternative architectures and configurations. Additionally, although the disclosure is described above in terms of various examples and aspects, it should be understood that the various features and functionality described in one or more of the individual examples are not limited in their applicability to the particular example with which they are described. They instead can be applied, alone or in some combination, to one or more of the other examples of the disclosure, whether or not such examples are described, and whether or not such features are presented as being a part of a described example. Thus the breadth and scope of the present disclosure should not be limited by any of the above-described example examples.

All references cited herein are incorporated herein by reference in their entirety. To the extent publications and patents or patent applications incorporated by reference contradict the disclosure contained in the specification, the specification is intended to supersede and/or take precedence over any such contradictory material.

Unless otherwise defined, all terms (including technical and scientific terms) are to be given their ordinary and customary meaning to a person of ordinary skill in the art, and are not to be limited to a special or customized meaning unless expressly so defined herein.

Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide example instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular example of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

The term "comprising as used herein is synonymous with "including," "containing," or "characterized by" and is inclusive or open-ended and does not exclude additional, unrecited elements or method steps.

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific examples and examples described herein, but rather to also cover all modification and alternatives coming with the true scope and spirit of the invention.

## REFERENCES

The following documents are incorporated herein by reference in their entirety:

[1] M. Vettoretti, G. Cappon, G. Acciaroli, A. Facchinetti, and G. Sparacino, "Continuous Glucose Monitoring: Current Use in Diabetes Management and Possible Future Applications," Journal of Diabetes Science and Technology, vol. 12, no. 5, pp. 1064-1071, 2018.

[2] B. McAdams and A. Rizvi, "An Overview of Insulin Pumps and Glucose Sensors for the Generalist," Journal of Clinical Medicine, vol. 5, no. 1, p. 5, 2016.

[3] I. Contreras and J. Vehi, "Artificial intelligence for diabetes management and decision support: Literature review," Journal of Medical Internet Research, vol. 20, no. 5, 2018.

[4] C. Luca, C. Giacomo, S. Giovanni, and F. Andrea, "A new integrated platform for gathering and managing multivariable and multisensor data in diabetes clinical studies," in 2020 International Conference on e- Health and Bioengineering (EHB), 2020, pp. 1-4.

[5] K. Turksoy, J. Kilkus, I. Hajizadeh, S. Samadi, J. Feng, M. Sevil, C. Lazaro, N. Frantz, E. Littlejohn, and A. Cinar, "Hypoglycemia Detec- tion and Carbohydrate Suggestion in an Artificial Pancreas," Journal of Diabetes Science and Technology, vol. 10, no. 6, pp. 1236-1244, 2016.

[6] N. Camerlingo, M. Vettoretti, S. Del Favero, G. Cappon, G. Sparacino, and A. Facchinetti, "A real-time continuous glucose monitoring-based algorithm to trigger hypotreatments to prevent/mitigate hypoglycemic events," Diabetes Technology and Therapeutics, vol. 21, no. 11, pp. 644- 655, 2019.

[7] J. P. Shivers, L. Mackowiak, H. Anhalt, and H. Zisser, "Turn It Off!": Diabetes Device Alarm Fatigue Considerations for the Present and the Future," Journal of Diabetes Science and Technology, vol. 7, no. 3, pp. 789-794, 2013.

[8] G. McGarraugh, "Alarm characterization for continuous glucose monitors used as adjuncts to self-monitoring of blood glucose," Journal of Diabetes Science and Technology, vol. 4, no. 1, pp. 41-48, 2010.

[9] X. Mo, Y. Wang, and X. Wu, "Hypoglycemia prediction using extreme learning machine (ELM) and regularized ELM," 2013 25th Chinese Control and Decision Conference, CCDC 2013, pp. 4405-4409, 2013.

[10] K. Turksoy, E. S. Bayrak, L. Quinn, E. Littlejohn, D. Rollins, and A. Cinar, "Hypoglycemia early alarm systems based on multivariable models," Industrial and Engineering Chemistry Research, vol. 52, no. 35, pp. 12 329-12 336, 2013.

[11] S. Oviedo, J. Veh'1, R. Calm, and J. Armengol, "A review of personalized blood glucose prediction strategies for T1DM patients," International Journal for Numerical Methods in Biomedical Engineering, vol. 33, no. 6, pp. 1-21, 2017.

[12] [12] O. Mujahid, I. Contreras, and J. Vehi, "Machine learning techniques for hypoglycemia prediction: Trends and challenges," Sensors (Switzerland), vol. 21, no. 2, pp. 1-21, 2021.

[13] H. Efendic, H. Kirchsteiger, G. Freckmann, and L. Del Re, "Short-term prediction of blood glucose concentration using interval probabilistic models," 2014 22nd Mediterranean Conference on Control and Automation, MED 2014, pp. 1494-1499, 2014.

[14] J. Yang, L. Li, Y. Shi, and X. Xie, "An ARIMA Model with Adaptive Orders for Predicting Blood Glucose Concentrations and Hypoglycemia," IEEE Journal of Biomedical and Health Informatics, vol. 23, no. 3, pp. 1251-1260, 2019.

[15] M. Eren-Oruklu, A. Cinar, D. K. Rollins, and L. Quinn, "Adaptive system identification for estimating future glucose concentrations and hypoglycemia alarms," Automatica, vol. 48, no. 8, pp. 1892-1897, 2012.

[16] M. Eren-Oruklu, A. Cinar, and L. Quinn, "Hypoglycemia prediction with subject-specific recursive time-series models," Journal of Diabetes Science and Technology, vol. 4, no. 1, pp. 25-33, 2010.

[17] C. Toffanin, S. Del Favero, E. M. Aiello, M. Messori, C. Cobelli, and L. Magni, "Glucose-insulin model identified in free-living conditions for hypoglycaemia prevention," Journal of Process Control, vol. 64, pp. 27-36, 2018.

[18] J. Veh'1, I. Contreras, S. Oviedo, L. Biagi, and A. Bertachi, "Prediction and prevention of hypoglycaemic events in type-1 diabetic patients using machine learning," Health informatics Journal, vol. 26, no. 1, pp. 703-718, 2020.

[19] D. Dave, D. J. DeSalvo, B. Haridas, S. McKay, A. Shenoy, C. J. Koh, M. Lawley, and M. Erraguntla, "Feature-Based Machine Learning Model for Real-Time Hypoglycemia Prediction," Journal of Diabetes Science and Technology, 2020.

[20] M. Gadaleta, A. Facchinetti, E. Grisan, and M. Rossi, "Prediction of Adverse Glycemic Events From Continuous Glucose Monitoring Signal," IEEE Journal of Biomedical and Health Informatics, vol. 23, no. 2, pp. 650-659, 2019.

[21] M. Frandes, B. Timar, and D. Lungeanu, "A risk based neural network approach for predictive modeling of blood glucose dynamics," Studies in Health Technology and Informatics, vol. 228, pp. 577-581, 2017.

[22] K. S. Eljil, G. Qadah, and M. Pasquier, "Predicting hypoglycemia in diabetic patients using data mining techniques," 2013 9th International Conference on Innovations in Information Technology, IIT 2013, pp. 130-135, 2013.

[23] E. I. Georga, V. C. Protopappas, D. Ardig'o, D. Polyzos, and D. I. Fotiadis, "A glucose model based on support vector regression for the prediction of hypoglycemic events under free-living conditions," Diabetes Technology and Therapeutics, vol. 15, no. 8, pp. 634-643, 2013.

[24] E. Daskalaki, K. Nørgaard, T. Züger, A. Prountzou, P. Diem, and S. Mougiakakou, "An early warning system for hypo- glycemic/hyperglycemic events based on fusion of adaptive prediction models," Journal of Diabetes Science and Technology, vol. 7, no. 3, pp. 689-698, 2013.

[25] L. Ljung, System identification - Theory for the user. PTR Prentice Hall, 1987.

[26] P. J. Kim and L. Huh, Kalman Filter for Beginners: with MATLAB Examples. MathWorks, 2011.

[27] C. Dalla Man, R. A. Rizza, and C. Cobelli, "Meal simulation model of the glucose-insulin system," IEEE Transactions on Biomedical Engineering, vol. 54, no. 10, pp. 1740-1749, 2007.

[28] D. A. Finan, H. Zisser, L. Jovanovic, W. C. Bevier, and D. E. Seborg, "Identification of Linear Dynamic Models for Type 1 Diabetes: a Simulation Study," in IFAC Proceedings Volumes, vol. 39, no. 2. IFAC, 2006, pp. 503-508.

[29] M. Messori, M. Ellis, C. Cobelli, P. D. Christofides, and L. Magni, "Improved postprandial glucose control with a customized Model Predictive Controller," Proceedings of the American Control Conference, pp. 5108-5115, 2015.

[30] F. Prendin, S. Del Favero, M. Vettoretti, G. Sparacino, and A. Facchinetti, "Forecasting of glucose levels and hypoglycemic events: Head-to-head comparison of linear and nonlinear data- driven algorithms based on continuous glucose monitoring data only," Sensors, vol. 21, no. 5, 2021.

[31] S. Faccioli, A. Facchinetti, G. Sparacino, G. Pillonetto, and S. Del Favero, "Linear Model Identification for Personalized Prediction and Control in Diabetes," IEEE Trans Biomed Eng. Online ahead of print, 2021, pMID: 34347589.

[32] S. Del Favero, A. Facchinetti, and C. Cobelli, "A Glucose-Specific Metric to Assess Predictors and Identify Models," Journal of Diabetes Science and Technology, vol. 6 (2), p. A30, 2012.

[33] B. Kovatchev, P. Cheng, S. M. Anderson, J. E. Pinsker, F. Boscari, B. A. Buckingham, F. J. Doyle, K. K. Hood, S. A. Brown, M. D. Breton, D. Chernavvsky, W. C. Bevier, P. K. Bradley, D. Bruttomesso, S. Del Favero, R. Calore, C. Cobelli, A. Avogaro, T. T. Ly, S. Shanmugham, E. Dassau, C. Kollman, J. W. Lum, and R. W. Beck, "Feasibility of Long-Term Closed-Loop Control: A Multicenter 6-Month Trial of 24/7 Automated Insulin Delivery," Diabetes Technology and Therapeutics, vol. 19, no. 1, pp. 18-24, 2017.

[34] P. Keith-Hynes, B. Mize, A. Robert, and J. Place, "The diabetes assistant: A smartphone-based system for real-time control of blood glucose," Electronics (Switzerland), vol. 3, no. 4, pp. 609-623, 2014.

[35] S. Bittanti, Model Identification and Data Analysis. John Wiley & Sons, Inc., 2019.

[36] W. L. Clarke, D. Cox, L. A. Gonder-Frederick, W. Carter, and S. L. Pohl, "Evaluating clinical accuracy of systems for self-monitoring of blood glucose," Diabetes Care, vol. 10, no. 5, pp. 622-628, 1987.

[37] C. P'erez-Gand'ia, A. Facchinetti, G. Sparacino, C. Cobelli, E. G'omez, M. Rigla, A. de Leiva, and M. Hernando, "Artificial neural network algorithm for online glucose prediction from continuous glucose monitoring," Diabetes technology & therapeutics, vol. 12, no. 1, pp. 81-88, 2010.

[38] J. Daniels, P. Herrero, and P. Georgiou, "A Multitask Learning Approach to Personalised Blood Glucose Prediction," IEEE J Biomed Health Inform, 2021.

[39] T. Danne, R. Nimri, T. Battelino, R. M. Bergenstal, K. L. Close, J. H. DeVries, S. Garg, L. Heinemann, I. Hirsch, S. A. Amiel, R. Beck, E. Bosi, B. Buckingham, C. Cobelli, E. Dassau, F. J. Doyle, S. Heller, R. Hovorka, W. Jia, T. Jones, O. Kordonouri, B. Kovatchev, A. Kowalski, L. Laffel, D. Maahs, H. R. Murphy, K. Nørgaard, C. G. Parkin, E. Renard, B. Saboo, M. Scharf, W. V. Tamborlane, S. A. Weinzimer, and M. Phillip, "International consensus on use of continuous glucose monitoring," Diabetes Care, vol. 40, no. 12, pp. 1631-1640, 2017.

[40] G. F. Franklin, J. D. Powell, M. L. Workman et al., Digital control of dynamic systems. Addison-wesley Reading, MA, 1998, vol. 3.

The description can be further characterized by the following clauses:

1. A method for managing blood glucose levels of a user, the method comprising:

receiving, by a computing device, a glucose measurement of the user from a sensor;
receiving, by the computing device, one or more values for one or more additional inputs relating to the blood glucose levels of the user;
processing, by the computing device, the glucose measurement and one or more values for the one or more additional inputs to obtain a plurality of predicted glucose values, each predicted glucose value of the plurality of predicted glucose values corresponding to a different prediction horizon and having a corresponding uncertainty;
generating, by the computing device, a confidence interval for each predicted glucose value of the plurality of predicted glucose values based on the each predicted glucose value and the corresponding uncertainty; and
generating, by the computing device, a decision support output in response to determining that the confidence intervals meet a threshold condition.

2. The method of clause 1, wherein the one or more additional inputs include meal intake information.

3. The method of clause 1, wherein the one or more additional inputs include an amount of exogenous insulin infused into the user.

4. The method of clause 1, wherein the one or more additional inputs include meal intake information and an amount of exogenous insulin administered to the user.

5. The method of clause 1, wherein the glucose measurement and one or more values are processed using one or more machine learning models.

6. The method of clause 5, wherein the one or more machine learning models comprise a predictive filter.

7. The method of clause 6, wherein the predictive filter is a Kalman filter.

8. The method of clause 7, wherein the Kalman filter is based on a predictive machine learning model trained to using a glucose-specific mean squared error (gMSE) loss function.

9. The method of clause 8, wherein the predictive machine learning model is an autoregressive integrated moving average with exogenous input (ARIMAX) machine learning model.

10. The method of clause 1, wherein the threshold condition is a minimum number of the confidence intervals being either (a) entirely above a hyperglycemic threshold or (b) entirely below a hypoglycemic threshold.

11. The method of clause 10, wherein generating the confidence interval, for each predicted glucose value, based on the each predicted glucose value and the corresponding uncertainty of the each predicted glucose value comprises scaling the uncertainty.

12. The method of clause 1, wherein the decision support output comprises a human-perceptible alert.

13. The method of clause 1, wherein the decision support output comprises a signal for transmission to an insulin delivery device to cause the insulin delivery device to alter an amount of insulin infused into the user.

14. The method of clause 1, wherein the decision support output comprises a human-perceptible instruction to consume carbohydrates.

15. A glucose monitoring system for managing blood glucose level of a user, the glucose monitoring system comprising:

a glucose sensor system configured to generate one or more glucose measurements for the user;
one or more processing devices;
one or more memory devices coupled to the one or more processing devices, the one or more memory devices storing executable code that, when executed by the one or more processing devices, causes the one or more processing devices to execute a method comprising:

receiving, for a time t(k), a glucose measurement g(k) of the one or more measurements from the glucose sensor system;

receiving meal intake information CHO(k) and an amount of infused insulin I(k) for the user;

processing g(k), CHO(k), and I(k) to obtain a plurality of predicted glucose values $\hat{g}(k+1|k)$ to $\hat{g}(k+PH_{max}|k)$, where $PH_{max}$ is an integer greater than one, and a plurality of uncertainties $\sigma^2(k+1|k)$ to $\sigma^2(k+PH_{max}|k)$;

generating a prediction funnel by calculating a plurality of confidence intervals $\hat{g}(k+i|k) \pm m*\sigma(k+i|k)$, i = 1 to $PH_{max}$, where m is a predetermined parameter; and

generating a decision support output in response to determining that the confidence intervals meet a threshold condition.

16. The glucose monitoring system of clause 15, wherein the glucose sensor system is a continuous glucose monitor.

17. The glucose monitoring system of clause 15, wherein the g(k), CHO(k), and I(k) are processed using one or more machine learning models comprising a Kalman filter.

18. The glucose monitoring system of clause 17, wherein the Kalman filter is configured based on a predictive machine learning model trained using a glucose-specific mean squared error (gMSE) loss function.

19. The glucose monitoring system of clause 15, wherein the threshold condition is a minimum number of the confidence intervals being either (a) entirely above a hyperglycemic threshold or (b) entirely below a hypoglycemic threshold.

20. The glucose monitoring system of clause 15, wherein the decision support output comprises at least one of:

a human-perceptible alert;

an instruction to alter an amount of insulin infused into the user; or

a human-perceptible instruction to consume carbohydrates.

## Claims

1. A method for managing blood glucose levels of a user, the method comprising:

receiving, by a computing device, a glucose measurement of the user from a sensor;

receiving, by the computing device, one or more values for one or more additional inputs relating to the blood glucose levels of the user;

processing, by the computing device, the glucose measurement and one or more values for the one or more additional inputs to obtain a plurality of predicted glucose values, each predicted glucose value of the plurality of predicted glucose values corresponding to a different prediction horizon and having a corresponding uncertainty;

generating, by the computing device, a confidence interval for each predicted glucose value of the plurality of predicted glucose values based on the each predicted glucose value and the corresponding uncertainty; and

generating, by the computing device, a decision support output in response to determining that the confidence intervals meet a threshold condition.

2. The method of claim 1, wherein the one or more additional inputs include meal intake information.

3. The method of claim 1, wherein the one or more additional inputs include an amount of exogenous insulin infused into the user.

4. The method of claim 1, wherein the one or more additional inputs include meal intake information and an amount of exogenous insulin administered to the user.

5. The method of claim 1, wherein the glucose measurement and one or more values are processed using one or more machine learning models.

6. The method of claim 5, wherein the one or more machine learning models comprise a predictive filter.

7. The method of claim 6, wherein the predictive filter is a Kalman filter.

8. The method of claim 7, wherein the Kalman filter is based on a predictive machine learning model trained to using a glucose-specific mean squared error (gMSE) loss function.

9. The method of claim 8, wherein the predictive machine learning model is an autoregressive integrated moving average with exogenous input (ARIMAX) machine learning model.

10. The method of claim 1, wherein the threshold condition is a minimum number of the confidence intervals being either (a) entirely above a hyperglycemic threshold or (b) entirely below a hypoglycemic threshold.

11. The method of claim 10, wherein the minimum number is a tunable value.

12. The method of claim 10, wherein generating the confidence interval, for each predicted glucose value, based on the each predicted glucose value and the corresponding uncertainty of the each predicted glucose value comprises scaling the uncertainty.

13. The method of claim 12, wherein the scaling is by a configurable parameter.

14. The method of claim 1, wherein the decision support output comprises a human-perceptible alert or alarm, a signal for transmission to an insulin delivery device to cause the insulin delivery device to alter an amount of insulin infused into the user or a human-perceptible instruction to consume carbohydrates.

15. A glucose monitoring system for managing blood glucose level of a user, the glucose monitoring system comprising:

a glucose sensor system configured to generate one or more glucose measurements for the user;
one or more processing devices;
one or more memory devices coupled to the one or more processing devices, the one or more memory devices storing executable code that, when executed by the one or more processing devices, causes the one or more processing devices to execute a method according to any of claims 1 to 14.

FIG. 1

MEDICAL DEVICE 208

CONTINUOUS ANALYTE SENSOR(S) 202

SENSOR ELECTRONICS MODULE 204

NON-ANALYTE SENSOR(S) 206

104

200

240

242

212

210

222

220

232

230

FIG. 2

300

INPUTS 128

Glucose (g(k))

Meal Intake (CHO(k))

Exogenous Insulin (I(k))

106

107

Prediction Module 116

Decision Support Engine 114

Decision Support Output

Alert 130

FIG. 3

FIG. 4

FIG. 5A

FIG. 5B

FIG. 5C

EP 4 685 813 A2

FIG. 6

FIG. 7

FIG. 8

FIG. 9

User Database
110

Historical
Records
Database 112

To/From Data
Communications Network

I/O Devices
1035

I/O Interface
1020

Processor
1005

Network Interface
1025

Interconnect (Bus)
1030

Memory
1010

Prediction Module 116

Decision Support Engine 114

Storage
1015

User Profile 118

Computing Device 1000

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63263433
- US 6001067 A
- US 20110027127 A1
- US 20060020187 A1
- US 20090137887 A1
- US 20070027385 A1

**Non-patent literature cited in the description**

- **M. VETTORETTI** ; **G. CAPPON** ; **G. ACCIAROLI** ; **A. FACCHINETTI** ; **G. SPARACINO**. Continuous Glucose Monitoring: Current Use in Diabetes Management and Possible Future Applications. *Journal of Diabetes Science and Technology*, 2018, vol. 12 (5), 1064-1071
- **B. MCADAMS** ; **A. RIZVI**. An Overview of Insulin Pumps and Glucose Sensors for the Generalist. *Journal of Clinical Medicine*, 2016, vol. 5 (1), 5
- **I. CONTRERAS** ; **J. VEHI**. Artificial intelligence for diabetes management and decision support: Literature review. *Journal of Medical Internet Research*, vol. 20 (5), 2018
- **C. LUCA** ; **C. GIACOMO** ; **S. GIOVANNI** ; **F. ANDREA**. A new integrated platform for gathering and managing multivariable and multisensor data in diabetes clinical studies. *2020 International Conference on e- Health and Bioengineering (EHB)*, 2020, 1-4
- **C. LAZARO** ; **N. FRANTZ** ; **E. LITTLEJOHN** ; **A. CINAR**. Hypoglycemia Detec- tion and Carbohydrate Suggestion in an Artificial Pancreas. *Journal of Diabetes Science and Technology*, 2016, vol. 10 (6), 1236-1244
- **N. CAMERLINGO** ; **M. VETTORETTI** ; **S. DEL FAVERO** ; **G. CAPPON** ; **G. SPARACINO** ; **A. FACCHINETTI**. A real-time continuous glucose monitoring-based algorithm to trigger hypotreatments to prevent/mitigate hypoglycemic events. *Diabetes Technology and Therapeutics*, 2019, vol. 21 (11), 644-655
- **J. P. SHIVERS** ; **L. MACKOWIAK** ; **H. ANHALT** ; **H. ZISSER**. Turn It Off!": Diabetes Device Alarm Fatigue Considerations for the Present and the Future. *Journal of Diabetes Science and Technology*, 2013, vol. 7 (3), 789-794
- **G. MCGARRAUGH**. Alarm characterization for continuous glucose monitors used as adjuncts to self-monitoring of blood glucose. *Journal of Diabetes Science and Technology*, 2010, vol. 4 (1), 41-48
- **X. MO** ; **Y. WANG** ; **X. WU**. Hypoglycemia prediction using extreme learning machine (ELM) and regularized ELM. *2013 25th Chinese Control and Decision Conference, CCDC 2013*, 2013, 4405-4409
- **K. TURKSOY** ; **E. S. BAYRAK** ; **L. QUINN** ; **E. LITTLEJOHN** ; **D. ROLLINS** ; **A. CINAR**. Hypoglycemia early alarm systems based on multivariable models. *Industrial and Engineering Chemistry Research*, 2013, vol. 52 (35), 12 329-12 336
- **S. OVIEDO** ; **J. VEH** ; **R. CALM** ; **J. ARMENGOL**. A review of personalized blood glucose prediction strategies for T1DM patients. *International Journal for Numerical Methods in Biomedical Engineering*, 2017, vol. 33 (6), 1-21
- **O. MUJAHID** ; **I. CONTRERAS** ; **J. VEHI**. Machine learning techniques for hypoglycemia prediction: Trends and challenges. *Sensors (Switzerland)*, 2021, vol. 21 (2), 1-21
- **H. EFENDIC** ; **H. KIRCHSTEIGER** ; **G. FRECK-MANN** ; **L. DEL RE**. Short-term prediction of blood glucose concentration using interval probabilistic models. *2014 22nd Mediterranean Conference on Control and Automation, MED 2014*, 2014, 1494-1499
- **J. YANG** ; **L. LI** ; **Y. SHI** ; **X. XIE**. An ARIMA Model with Adaptive Orders for Predicting Blood Glucose Concentrations and Hypoglycemia. *IEEE Journal of Biomedical and Health Informatics*, 2019, vol. 23 (3), 1251-1260
- **M. EREN-ORUKLU** ; **A. CINAR** ; **D. K. ROLLINS** ; **L. QUINN**. Adaptive system identification for estimating future glucose concentrations and hypoglycemia alarms. *Automatica*, 2012, vol. 48 (8), 1892-1897
- **M. EREN-ORUKLU** ; **A. CINAR** ; **L. QUINN**. Hypoglycemia prediction with subject-specific recursive time-series models. *Journal of Diabetes Science and Technology*, 2010, vol. 4 (1), 25-33

- **C. TOFFANIN** ; **S. DEL FAVERO** ; **E. M. AIELLO** ; **M. MESSORI** ; **C. COBELLI** ; **L. MAGNI**. Glucose-insulin model identified in free-living conditions for hypoglycaemia prevention. *Journal of Process Control*, 2018, vol. 64, 27-36

- **J. VEH** ; **I. CONTRERAS** ; **S. OVIEDO** ; **L. BIAGI** ; **A. BERTACHI**. Prediction and prevention of hypoglycaemic events in type-1 diabetic patients using machine learning. *Health informatics Journal*, 2020, vol. 26 (1), 703-718

- **D. DAVE** ; **D. J. DESALVO** ; **B. HARIDAS** ; **S. MCKAY** ; **A. SHENOY** ; **C. J. KOH** ; **M. LAWLEY** ; **M. ERRAGUNTLA**. Feature-Based Machine Learning Model for Real-Time Hypoglycemia Prediction. *Journal of Diabetes Science and Technology*, 2020

- **M. GADALETA** ; **A. FACCHINETTI** ; **E. GRISAN** ; **M. ROSSI**. Prediction of Adverse Glycemic Events From Continuous Glucose Monitoring Signal. *IEEE Journal of Biomedical and Health Informatics*, 2019, vol. 23 (2), 650-659

- **M. FRANDES** ; **B. TIMAR** ; **D. LUNGEANU**. A risk based neural network approach for predictive modeling of blood glucose dynamics. *Studies in Health Technology and Informatics,*, 2017, vol. 228, 577-581

- **K. S. ELJIL** ; **G. QADAH** ; **M. PASQUIER**. Predicting hypoglycemia in diabetic patients using data mining techniques. *2013 9th International Conference on Innovations in Information Technology, IIT 2013*, 2013, 130-135

- **E. I. GEORGA** ; **V. C. PROTOPAPPAS** ; **D. ARDIG'O** ; **D. POLYZOS** ; **D. I. FOTIADIS**. A glucose model based on support vector regression for the prediction of hypoglycemic events under free-living conditions. *Diabetes Technology and Therapeutics*, 2013, vol. 15 (8), 634-643

- **E. DASKALAKI** ; **K. NØRGAARD** ; **T. ZÜGER** ; **A. PROUNTZOU** ; **P. DIEM** ; **S. MOUGIAKAKOU**. An early warning system for hypo- glycemic/hyperglycemic events based on fusion of adaptive prediction models. *Journal of Diabetes Science and Technology*, 2013, vol. 7 (3)

- **L. LJUNG**. System identification - Theory for the user. PTR Prentice Hall, 1987

- **P. J. KIM** ; **L. HUH**. Kalman Filter for Beginners: with MATLAB Examples. *MathWorks*, 2011

- **C. DALLA MAN** ; **R. A. RIZZA** ; **C. COBELLI**. Meal simulation model of the glucose-insulin system. *IEEE Transactions on Biomedical Engineering*, 2007, vol. 54 (10), 1740-1749

- **D. A. FINAN** ; **H. ZISSER** ; **L. JOVANOVIC** ; **W. C. BEVIER** ; **D. E. SEBORG**. Identification of Linear Dynamic Models for Type 1 Diabetes: a Simulation Study. *IFAC Proceedings Volumes*, 2006, vol. 39 (2), 503-508

- **M. MESSORI** ; **M. ELLIS** ; **C. COBELLI** ; **P. D. CHRISTOFIDES** ; **L. MAGNI**. Improved postprandial glucose control with a customized Model Predictive Controller. *Proceedings of the American Control Conference*, 2015, 5108-5115

- **F. PRENDIN** ; **S. DEL FAVERO** ; **M. VETTORETTI** ; **G. SPARACINO** ; **A. FACCHINETTI**. Forecasting of glucose levels and hypoglycemic events: Head-to-head comparison of linear and nonlinear data- driven algorithms based on continuous glucose monitoring data only. *Sensors*, vol. 21 (5), 2021

- **S. FACCIOLI** ; **A. FACCHINETTI** ; **G. SPARACINO** ; **G. PILLONETTO** ; **S. DEL FAVERO**. Linear Model Identification for Personalized Prediction and Control in Diabetes. *IEEE Trans Biomed Eng. Online ahead of print*, 2021

- **S. DEL FAVERO** ; **A. FACCHINETTI** ; **C. COBELLI**. A Glucose-Specific Metric to Assess Predictors and Identify Models. *Journal of Diabetes Science and Technology*, 2012, vol. 6 (2), A30

- **B. KOVATCHEV** ; **P. CHENG** ; **S. M. ANDERSON** ; **J. E. PINSKER** ; **F. BOSCARI** ; **B. A. BUCKINGHAM** ; **F. J. DOYLE** ; **K. K. HOOD** ; **S. A. BROWN** ; **M. D. BRETON**. Feasibility of Long-Term Closed-Loop Control: A Multicenter 6-Month Trial of 24/7 Automated Insulin Delivery. *Diabetes Technology and Therapeutics*, 2017, vol. 19 (1), 18-24

- **P. KEITH-HYNES** ; **B. MIZE** ; **A. ROBERT** ; **J. PLACE**. The diabetes assistant: A smartphone-based system for real-time control of blood glucose. *Electronics (Switzerland)*, 2014, vol. 3 (4), 609-623

- **S. BITTANTI**. Model Identification and Data Analysis. John Wiley & Sons, Inc., 2019

- **W. L. CLARKE** ; **D. COX** ; **L. A. GONDER-FREDERICK** ; **W. CARTER** ; **S. L. POHL**. Evaluating clinical accuracy of systems for self-monitoring of blood glucose. *Diabetes Care*, 1987, vol. 10 (5), 622-628

- **C. P'EREZ-GAND'IA** ; **A. FACCHINETTI** ; **G. SPARACINO** ; **C. COBELLI** ; **E. G'OMEZ** ; **M. RIGLA** ; **A. DE LEIVA** ; **M. HERNANDO**. Artificial neural network algorithm for online glucose prediction from continuous glucose monitoring. *Diabetes technology & therapeutics*, 2010, vol. 12 (1), 81-88

- **J. DANIELS** ; **P. HERRERO** ; **P. GEORGIOU**. A Multitask Learning Approach to Personalised Blood Glucose Prediction. *IEEE J Biomed Health Inform*, 2021

- **T. DANNE** ; **R. NIMRI** ; **T. BATTELINO** ; **R. M. BERGENSTAL** ; **K. L. CLOSE** ; **J. H. DEVRIES** ; **S. GARG** ; **L. HEINEMANN** ; **I. HIRSCH** ; **S. A. AMIEL**. International consensus on use of continuous glucose monitoring. *Diabetes Care*, 2017, vol. 40 (12), 1631-1640

• **G. F. FRANKLIN** ; **J. D. POWELL** ; **M. L. WORKMAN et al.** Digital control of dynamic systems. *Addison-wesley Reading, MA*, 1998, vol. 3